# EUROPEAN PATENT SPECIFICATION

(11) **EP 3 370 758 B1**
(45) Date of publication and mention of the grant of the patent: **02.12.2026**
(21) Application number: 16861166.3
(22) Date of filing: 04.11.2016
(51) Int. Cl.: A61K 38/18, A61K 31/7008, A61K 31/737, A61K 9/08, A61P 19/00, A61P 19/02, C07H 5/06, C07K 14/475, C07K 14/495, C08B 37/08, A61K 9/00, A61K 47/20, A61K 47/26, A61K 47/36, A61K 47/38, C07H 3/02, C08L 1/28, C08L 5/08

(54) **COMPOSITIONS AND METHODS COMPRISING GROWTH FACTORS, CHONDROITIN AND GLUCOSAMINE FOR DEGENERATIVE DISC REGENERATION**
ZUSAMMENSETZUNGEN UND VERFAHREN MIT WACHSTUMSFAKTOREN, CHONDROITIN UND GLUCOSAMIN ZUR REGENERATION VON DEGENERATIVEN BANDSCHEIBEN
MÉTHODES ET COMPOSITIONS COMPRENANT DES FACTEURS DE CROISSANCES, DE LA CHONDROÏTINE ET DE LA GLUCOSAMINE POUR LA RÉGÉNÉRATION D'UN DISQUE DÉGÉNÉRATIF

(30) Priority: 06.11.2015 US 201562252234 P
(43) Date of publication of application: 12.09.2018
(73) Proprietor: University Health Network, Toronto, ON M5G 2C4 (CA); Notogen, Inc., Toronto, ON M5G 0B7 (CA)
(72) Inventor: ERWIN, William Mark, North York, Ontario M2M 2Y8 (CA); EEK, Björn C.J., Long Beach, CA 90807 (US)
(74) Representative: HGF
(86) International application number: PCT/CA2016/051291
(87) International publication number: WO 2017/075719

(56) References cited:
- WO-A1-2010/088775
- US-A1- 2007 254 040
- US-B2- 8 048 865
- VALENTINA CALAMIA ET AL: "A pharmacoproteomic study confirms the synergistic effect of chondroitin sulfate and glucosamine", SCIENTIFIC REPORTS, vol. 4, no. 5069, 10 June 2014 (2014-06-10), XP055588940, DOI: 10.1038/srep05069
- ERWIN, WM ET AL.: "Nucleus pulposus notochord cells secrete connective tissue growth factor and up-regulate proteoglycan expression by intervertebral disc chondrocytes ''.", ARTHRITIS AND RHEUMATISM, vol. 54, no. 12, December 2006 (2006-12-01), pages 3859 - 3867, XP 055382078, ISSN: 0004-3591
- ALI, AA ET AL.: "Oral glucosamine increases expression of transforming growth factor beta1 (TGFbeta1) and connective tissue growth factor (CTGF) mRNA in rat cartilage and kidney: Implications for human efficacy and toxicity''.", ARCHIVES OF BIOCHEMISTRY AND BIOPHYSICS, vol. 510, no. 1, June 2011 (2011-06-01), pages 11 - 18, XP 028207879, ISSN: 1096-0384
- CHO, H ET AL.: "Synergistic effect of combined growth factors in porcine intervertebral disc degeneration''.", CONNECTIVE TISSUE RESEARCH, vol. 54, no. 3, April 2013 (2013-04-01), pages 181 - 186, XP 055382079, ISSN: 1607-8438
- ERWIN, WM ET AL.: "Notochordal cells protect nucleus pulposus cells from degradation and apoptosis: implications for the mechanism of intervertebral disc degeneration ''.", ARTHRITIS RESEARCH AND THERAPY, vol. 13, no. 6, 29 December 2011 (2011-12-29), pages 1 - 15, XP 021094491, ISSN: 1478-6362
- YANG, H ET AL.: "TGF-beta1 suppresses inflammation in cell therapy for intervertebral disc degeneration ''.", SCI. REP., vol. 5, 20 August 2015 (2015-08-20), pages 13254, XP 055285476, ISSN: 2045-2322
- BACH, FC ET AL.: "The species-specific regenerative effects of notochordal cell -conditioned medium on chondrocyte-like cells derived from degenerated human intervertebral discs", EUR CELL MATER, vol. 30, 1 January 2015 (2015-01-01), pages 132 - 146, XP055540982, ISSN: 1473-2262
- MILLER, S ET AL.: "Notochordal cell conditioned medium (NCCM) regenerates end-stage human osteoarthritic articular chondrocytes and promotes a healthy phenotype''.", ARTHRITIS RESEARCH & THERAPY, vol. 18, no. 125, 2 June 2016 (2016-06-02), pages 1 - 11, XP 055382080, ISSN: 1478-6362

## Description

This application claims priority from United States Application No. 62/252,234 filed November 6, 2015.

### FIELD OF THE INVENTION

This invention relates generally to spinal disc degeneration, and more particularly to a composition suitable for intervertebral disc (IVD) injection, and suitably to a therapeutically effective amount of such a composition for use in the treatment of pain emanating from a spinal disc, for use in the treatment of degenerative disc disease, for use in the treatment or prevention of spinal disc injury, for use in the regeneration of a degenerative spinal disc or for use in preventing the progression of loss of spinal disc height in a degenerative or injured spinal disc.

### BACKGROUND OF THE INVENTION

Degenerative disc disease (DDD) is the predominant contributor (~40%) to the genesis of low back pain and is a major cause of disability worldwide, imposing enormous socio-economic burden and clinical costs to the society^{1,2}. The healthy intervertebral disc (IVD) is composed of a central proteoglycan rich nucleus pulposus (NP) surrounded by the concentric annulus fibrosus (AF) and attached to the adjacent vertebrae by thin cartilaginous end plates. In humans, large, vacuolated notochordal cells (NCs) present in the NP during childhood are gradually replaced by small chondrocyte-like cells (CLCs) by early adolescence³⁻⁵. Importantly, there is a temporal relationship between the loss of NCs and the onset of DDD in humans, where degeneration of the NP often leads to compromised disc function, impaired load bearing, associated pain and disability³⁻⁵. Currently there are no interventions capable of ameliorating the degenerative process or that can promote repair. In fact, surgical procedures such as spinal fusion may hasten adjacent segment degeneration⁶⁻⁸. Thus, the development of minimally invasive regenerative therapies is an attractive alternative for disc repair⁹⁻¹⁵.

Unlike humans, non-chondrodystrophic canines (NCD) preserve NCs within their NPs and are relatively resistant to DDD^{16,17}. Notochordal cell derived conditioned medium (NCCM) obtained from the non-chondrodystrophic canine (NCD) nucleus pulposus confers anabolic characteristics upon NP cells¹⁸⁻²⁰. Similarly, other studies have demonstrated increased proteoglycan synthesis and cell proliferation in NP cells treated with NCCM *in vitro*²¹⁻²⁴. The reason for apparent beneficial effects upon NCCM treatment has heretofore been unclear, however, and NCCM as a treatment per se has a number of disadvantages, notably including heterogeneity of the mixture.

US8048865B2 teaches injectable glycosaminoglycans for disc therapy, but does not mention or suggest adding CTGF or TGFβ1. US2007/254040A1 discloses a nutritional supplement for improving the conditions of bones or cartilage comprising glucosamine HCl, chondroitin sulfate and growth factors. The effect of glucosamine HCl and chondroitin sulfate has been studied for the treatment of osteoarthritis by Calamia et al. (Scientific Reports, 2014); while Erwin et al. (Arthritis & Rheumatology, 2006) have investigated the role of growth factors in the treatment of degenerative disc disease.

Therefore, there is a need for an improved treatment for disc degeneration.

### SUMMARY OF THE INVENTION

In a first aspect, a composition is provided comprising at least one glycosaminoglycan, or a pharmaceutically acceptable salt thereof; connective tissue growth factor (CTGF) present at a concentration between 50 ng/mg to 500 ng/mg of the composition; and transforming growth factor beta 1 (TGFβ1) present at a concentration between 1 ng/mL and 100 ng/mL of the composition; wherein the composition is suitable for intervertebral disc (IVD) injection.

In an embodiment, the composition of the first aspect of the invention comprises glucosamine or a pharmaceutically acceptable salt thereof, or chondroitin or a pharmaceutically acceptable salt thereof.

In another embodiment, the composition of the first aspect of the invention comprises glucosamine or a pharmaceutically acceptable salt thereof, and chondroitin or a pharmaceutically acceptable salt thereof.

In a second aspect, a composition is provided comprising chondroitin or a pharmaceutically acceptable salt thereof; glucosamine or a pharmaceutically acceptable salt thereof; and at least one growth factor selected from the group consisting of:
a) connective tissue growth factor (CTGF) present at a concentration between 50 ng/mg to 500 ng/mg of the composition; or
b) transforming growth factor beta 1 (TGFβ1) present at a concentration between 1 ng/mL and 100 ng/mL of the composition; or
c) CTGF present at a concentration between about 50 ng/mg to 500 ng/mg of the composition and TGFβ1 present at a concentration between 1 ng/mL and about 100 ng/mL of the composition;
wherein the composition is suitable for intervertebral disc (IVD) injection..

In an embodiment of the compositions provided herein, the chondroitin when present is chondroitin sulfate, optionally wherein the chondroitin sulfate is present at a level selected from the group consisting of 0.1% to 2.0% by weight of the composition, 0.5% to 2.0% by weight of the composition, 0.1% to 0.5% by weight of the composition, 0.1% by weight of the composition, and 0.5% by weight of the composition.

In an embodiment of the compositions provided herein, the glucosamine when present is glucosamine hydrochloride, optionally wherein the glucosamine hydrochloride is present at a level selected from the group consisting of 1% to 25% by weight of the composition, 5% to 20% by weight of the composition, 1% to 5% by weight of the composition, 1.0% to 1.5% by weight of the composition, 1% by weight of the composition, 1.5% by weight of the composition, 5% by weight of the composition, 20% by weight of the composition, and 25% by weight of the composition.

In an embodiment of the compositions provided herein, the CTGF when present is present at a concentration selected from the group consisting of: at least 50 ng/mL of the composition, at least 100 ng/mL of the composition, at least 200 ng/mL of the composition, between 50 ng/mg to 500 ng/mg of the composition, 50 ng/mL of the composition, 75 ng/mL of the composition, 100 ng/mL of the composition, 125 ng/mL of the composition, 150 ng/mL of the composition, 175 ng/mL of the composition, 200 ng/mL of the composition, 225 ng/mL of the composition, about 250 ng/mL of the composition, 275 ng/mL of the composition, 300 ng/mL of the composition, 325 ng/mL of the composition, 350 ng/mL of the composition, 375 ng/mL of the composition, 400 ng/mL of the composition, 425 ng/mL of the composition, 450 ng/mL of the composition, 475 ng/mL of the composition, and 500 ng/mL of the composition.

In an embodiment of the compositions provided herein, the TGFβ1 when present is present at a concentration selected from the group consisting of: at least 1 ng/mL of the composition, at least 5 ng/mL of the composition, at least 10 ng/mL of the composition, between 10 ng/mL and 100 ng/mL of the composition, between 10 ng/mg and 100 ng/mg of the composition, 1 ng/mL of the composition, 5 ng/mL of the composition, 10 ng/mL of the composition, 15 ng/mL of the composition, 20 ng/mL of the composition, 25 ng/mL of the composition, 30 ng/mL of the composition, 35 ng/mL of the composition, 40 ng/mL of the composition, 45 ng/mL of the composition, 50 ng/mL of the composition, 55 ng/mL of the composition, 60 ng/mL of the composition, 65 ng/mL of the composition, 70 ng/mL of the composition, 75 ng/mL of the composition, about 80 ng/mL of the composition, about 85 ng/mL of the composition, 90 ng/mL of the composition, 95 ng/mL of the composition, and 100 ng/mL of the composition.

In an embodiment, the compositions provided herein further comprise at least one sugar, preferably wherein the at least one sugar is dextrose or a pharmaceutically acceptable salt thereof, preferably wherein the dextrose or the pharmaceutically acceptable salt thereof is present at a level selected from the group consisting of up to 25% by weight of the composition, preferably 1% to 2% by weight of the composition. In an embodiment of the second aspect of the invention, the dextrose is present in about 1.0% to about 1.5% by weight of the composition.

In an embodiment of the second aspect of the invention, the composition further comprises at least one pharmaceutically acceptable carrier, optionally wherein the at least one pharmaceutically acceptable carrier is selected from the group consisting of carboxymethylcellulose and hyaluronic acid.

In an embodiment of the first aspect of the invention, the composition comprises carboxymethylcellulose.

In an embodiment of the first aspect of the invention, the composition comprises chondroitin, glucosamine and dextrose, and may optionally further comprise a carrier, optionally wherein the carrier is carboxymethylcellulose and/or hyaluronic acid and optionally wherein the carboxymethylcellulose is present at less than 0.5% by weight.

In an embodiment of the second aspect of the invention, the composition further comprises water.

In an embodiment of the second aspect of the invention, the composition is in the form of an aqueous solution, and may optionally further comprise dextrose, carboxymethylcellulose, dimethyl sulfoxide and/or hyaluronic acid.

In an embodiment of the second aspect of the invention, the composition further comprises a buffer, preferably wherein the buffer is present in a quantity sufficient to stabilize the composition pH between 6 and 7.

In an embodiment of the second aspect of the invention, the composition further comprises dimethyl sulfoxide.

In an embodiment of the second aspect of the invention, the composition further comprises an anesthetic, preferably wherein the anesthetic is bupivacaine.

In another aspect, provided herein is a therapeutically effective amount of a composition provided herein for use in a method of treating pain in a patient that emanates from a spinal disc, wherein the composition is for administration by injecting the composition into the pain emanating disc, or into an adjacent spinal disc, of the patient. Implementations may include one or more of the following. The pain emanating disc has degenerated. The pain emanating disc has been previously injured.

In another aspect, provided herein is a therapeutically effective amount of a composition provided herein for use in a method of treating degenerative disc disease (DDD) in a patient, wherein the composition is for administration by injecting into the degenerative disc, or into an adjacent spinal disc, of the patient.

In another aspect, provided herein is a therapeutically effective amount of a composition provided herein for use in in the treatment or prevention of spinal disc injury in a patient, wherein the composition is for administration by injecting into the injured spinal disc, or into an adjacent spinal disc, of the patient.

In an example not encompassed by the wording of the claims, a method of treating pain, arthritis, or suspected arthritis in a body area of a patient is described, the method including injecting a therapeutically effective amount of one of the compositions described above into the body area. Implementations may include one or more of the following: the body area is spine; the body area is leg; the body area is a joint: the body area is knee; the body area is a shoulder; the body area is an arm; the body area is an elbow; or the body area is a wrist.

In another aspect, provided herein is a therapeutically effective amount of a composition according to the second aspect of the invention for use in the regeneration of a degenerative spinal disc in a patient, wherein the composition is for administration by injecting the composition into the degenerative spinal disc, or an adjacent spinal disc, of the patient.

In another aspect, provided herein is a therapeutically effective amount of a composition of the invention, wherein the composition comprises glucosamine or a pharmaceutically acceptable salt thereof and chondroitin or a pharmaceutically acceptable salt thereof, for use in preventing the progression of loss of spinal disc height in a degenerative or injured spinal disc of a patient, which degenerative or injured spinal disc has lost disc height relative to a non-degenerative or uninjured spinal disc, wherein the composition is for administration by injecting the composition into the degenerative or injured spinal disc, or an adjacent spinal disc, of the patient.

In this respect, before explaining at least one embodiment of the invention in detail, it is to be understood that the invention is not limited in its application to the details of construction and to the arrangements of the components set forth in the following description or illustrated in the drawings. The invention is capable of other embodiments and of being practiced and carried out in various ways. Also, it is to be understood that the phraseology and terminology employed herein are for the purpose of description and should not be regarded as limiting.

### BRIEF DESCRIPTION OF THE DRAWINGS

In the drawings, embodiments of the invention are illustrated by way of example. It is to be expressly understood that the description and drawings are only for the purpose of illustration and as an aid to understanding, and are not intended as a definition of the limits of the invention.

Embodiments will now be described, by way of example only, with reference to the attached figures, wherein:

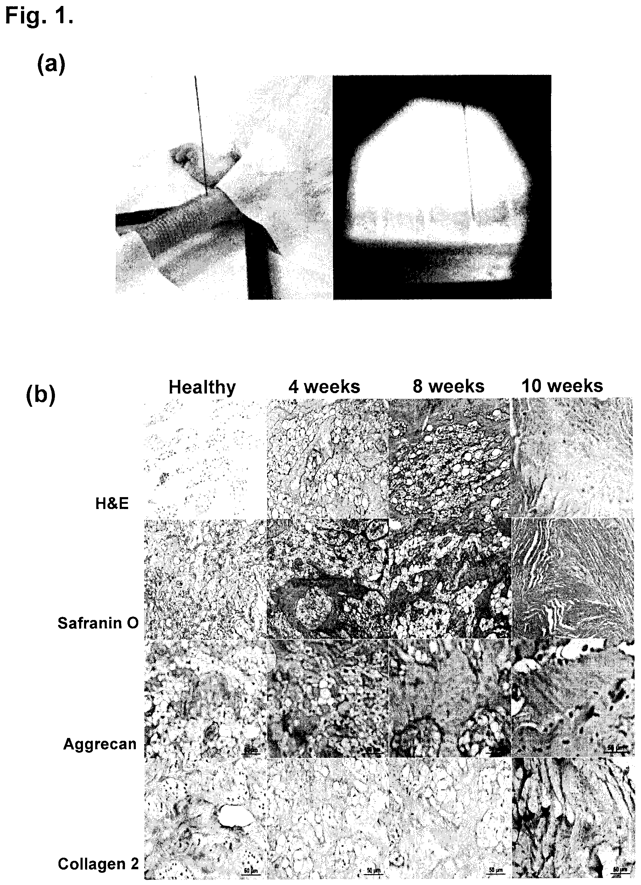

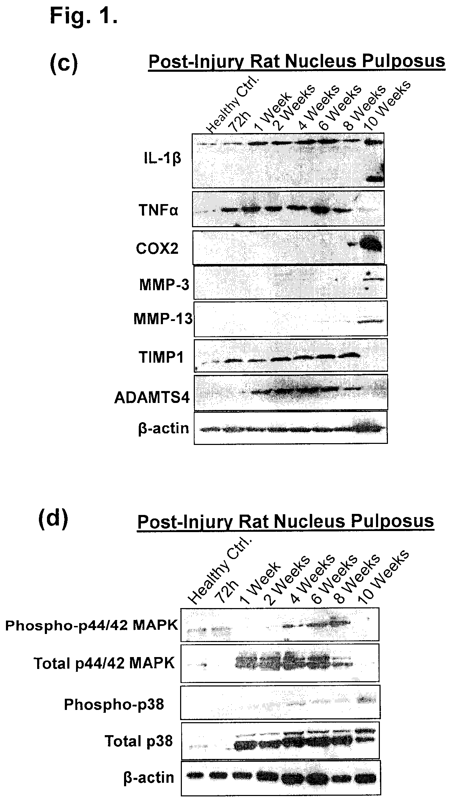

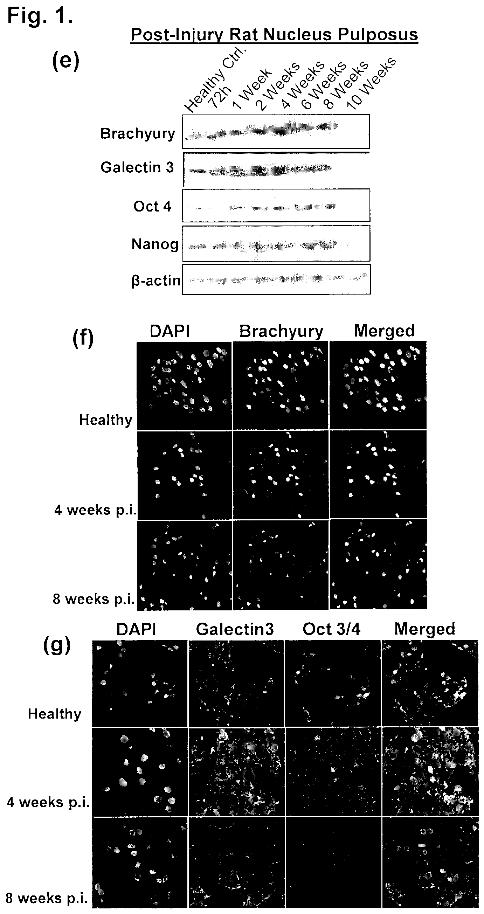

**FIG. 1** shows Needle puncture injury in rat-tail disc leads to the development of fibrocartilaginous matrix and loss of notochordal (NC) and stem cells in nucleus pulposus (NP). (**a**) Fluoroscopic image-guided needle puncture injury in rat-tail disc NP. (**b**) Histological analysis (H&E) and Safranin O staining showing development of a fibrocartilaginous matrix over a period of 10 weeks post-injury in rat NP. Immunohistochemistry showing the loss of the ECM proteins, aggrecan and collagen 2 in time dependent manner (healthy to 10 weeks post-injury, Scale bar 50µ). (**c**) Western blot showing alterations in the expression of pro-inflammatory cytokines (IL-1β and TNFα), inflammation mediator, Cox2 and ECM proteins (MMP-3, MMP-13, TIMP1, ADAMTS4) in a time dependent manner in post-injury rat NP tissue lysates. (**d**) Western blot of phospho-p42/44 (Thr202 / Tyr204), total - p42/44, phospho-p38MAPK (Thr180 / Tyr182) and total p38MAPK in tissue lysates obtained from rat tail injured disc NP. (**e**) Western blot analysis showing loss of NC markers (brachyury, galectin 3) and stem cell markers (Oct4, Nanog) in NPs obtained from rat-tail injured discs over a period of 10 weeks. β-actin was used a loading control in western blots. Immunofluorescence verifying the decrease in (f) nuclear brachyury (**g**) galectin 3 (membrane/cytoplasm) and nuclear Oct4 expression in rat tail injured discs NPs as compared to healthy control disc NP (Scale bar 10µ).

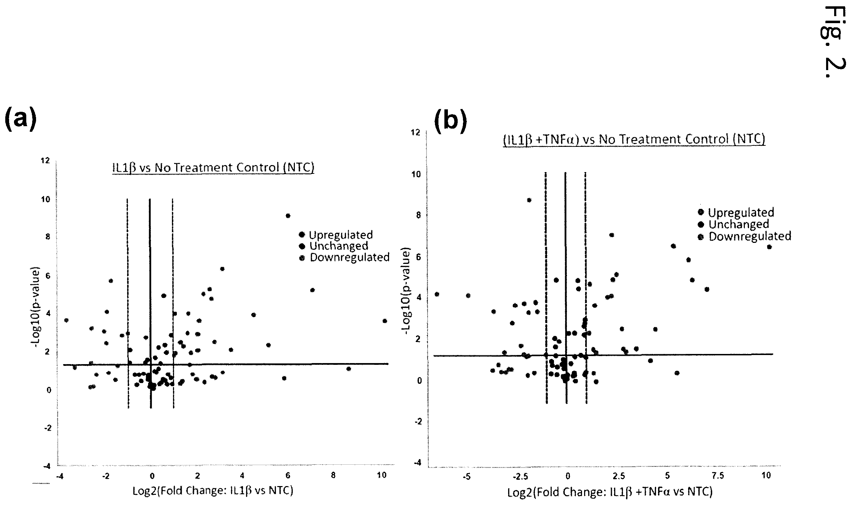

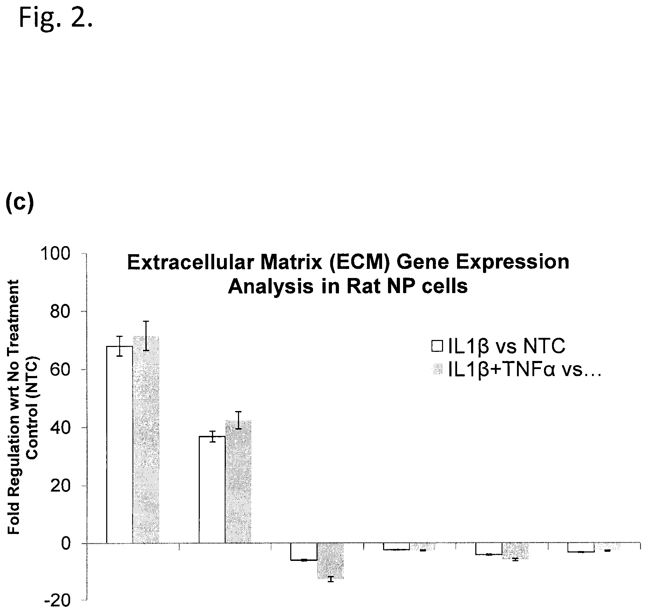

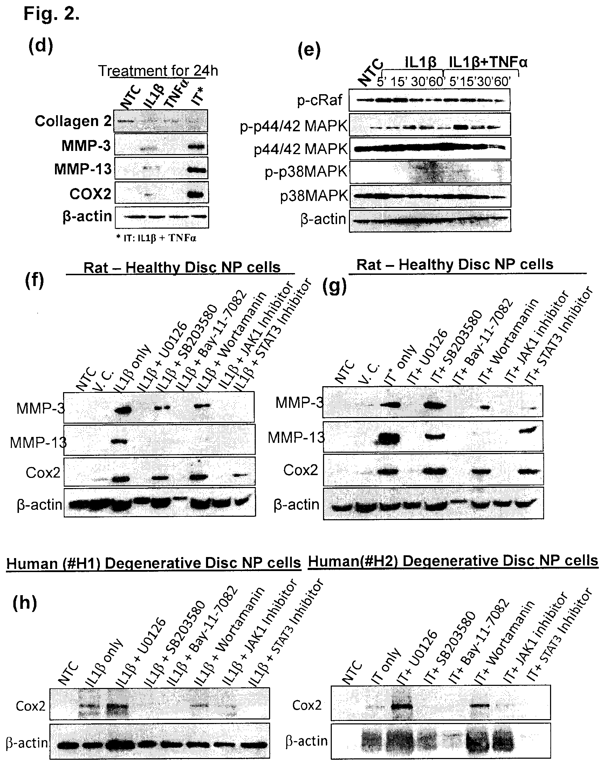

**FIG. 2** shows IL-1β plays a role in NP - ECM degradation in DDD. Volcano plots depicting differential expression of ECM genes on treatment with (**a**) IL-1β alone or (**b**) combination of IL-1β and TNFα, as compared to no treatment controls (NTC) in ECM gene array (n=3,p<0.05). (**c**) Representative histograms showing significant differential expression (p < 0.05) of ECM genes in healthy rat NP cells treated with IL-1β alone, or in combination with TNFα for 24 hrs. (**d**) Western blots showing IL-1β and TNFα reduced collagen 2, but induced expression of matrix metalloproteinases (MMP-3, MMP-13) and the inflammation mediator, Cox-2 in NP cells obtained from healthy rat IVD - NP. (**e**) Western blot of phospho-cRaf (Ser259), phospho-p42/44 (Thr202 / Tyr204), total - p42/44, phospho-p38MAPK (Thr180 / Tyr182) and total-p38MAPK in healthy rat NP cells treated with IL-1β alone or in combination with TNFα for 5 - 60 minutes. Western blot of MMP-3, MMP-13 and Cox2 in cell lysates obtained from healthy rat NP cells treated with (**f**) IL-1β alone, (**g**) combination of IL-1β and TNFα in presence of specific inhibitors targeting p42/44 (U0126), p38MAPK (SB203580), NFκB (BAY-11-7082), PI3K (Wortamanin), Jak1 and STAT3 (WP1066). (**h**) Western blots showing decreased expression of cytokine induced Cox2 in presence of SB203580, BAY-11-7082, Jak1 and STAT3 (WP1066) inhibitor in human degenerative disc NP cells.

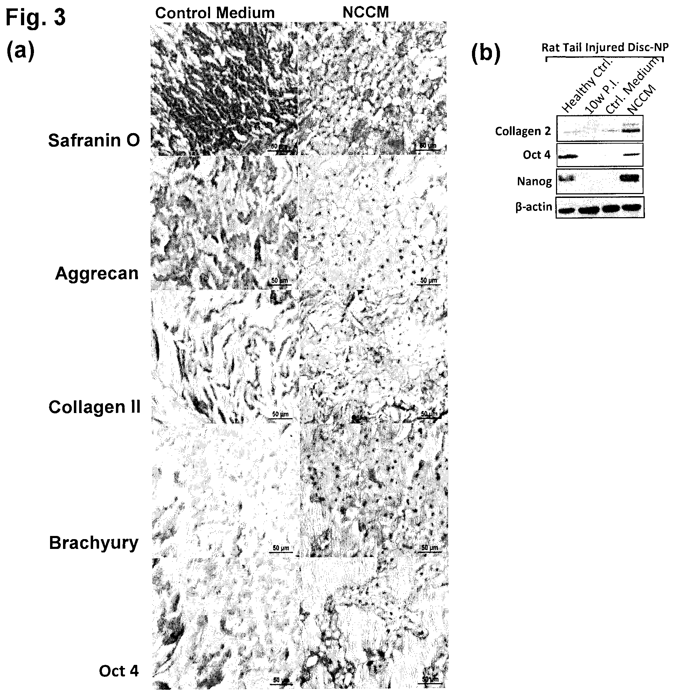

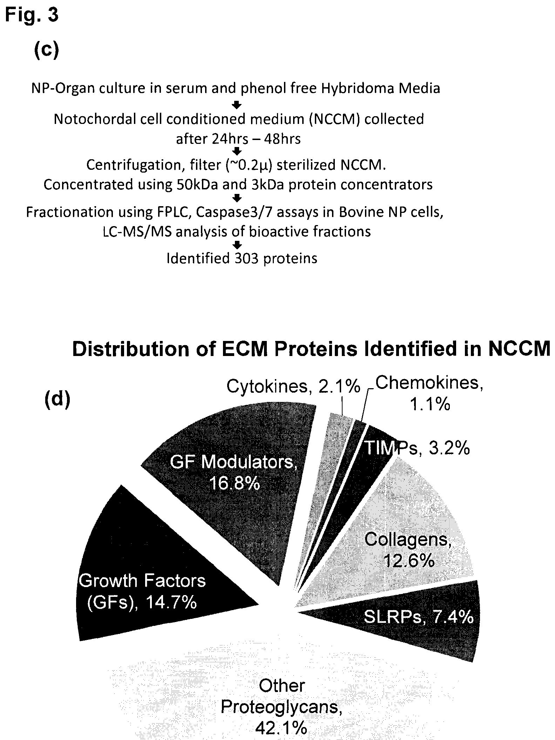

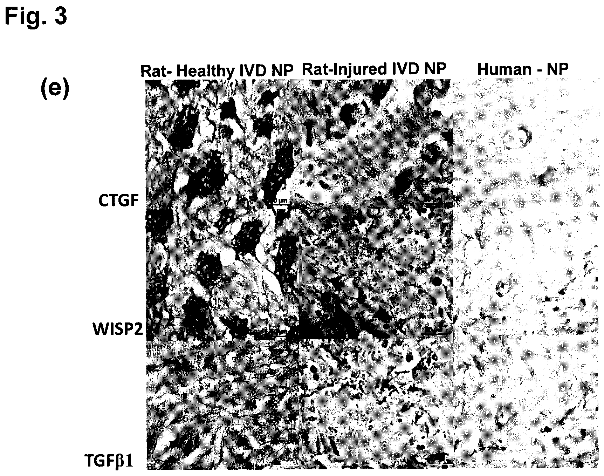

**FIG. 3** shows NCCM confers anabolic and anti-catabolic characteristics to degenerating NP in rat-tail injured IVD - NP. (**a**) Safranin O staining showing alteration from the normal plentiful and large notochordal cell, proteoglycan-rich ECM to one largely devoid of notochordal cells replaced by small NP cells. Immunohistochemistry of aggrecan, collagen 2, brachyury and Oct4 in paraffin embedded sections of rat-tail injured discs treated with protein free Hybridoma medium used as control or NCCM (Scale bar 50µ). (**b**) Western blot of collagen 2 and the stem cell markers Oct4 and Nanog in tissue lysates obtained from rat-tail injured disc NPs and healthy controls. (**c**) Schematic representation of the methodology for identification of proteins in NCCM using mass - spectroscopy. (**d**) Pie-chart showing distribution of ECM proteins identified in NCCM. (**e**) Immunohistochemistry showing expression of CTGF, WISP-2 and TGFβ1 in paraffin-embedded sections of rat NP (healthy and injured discs) and human degenerative disc NP.

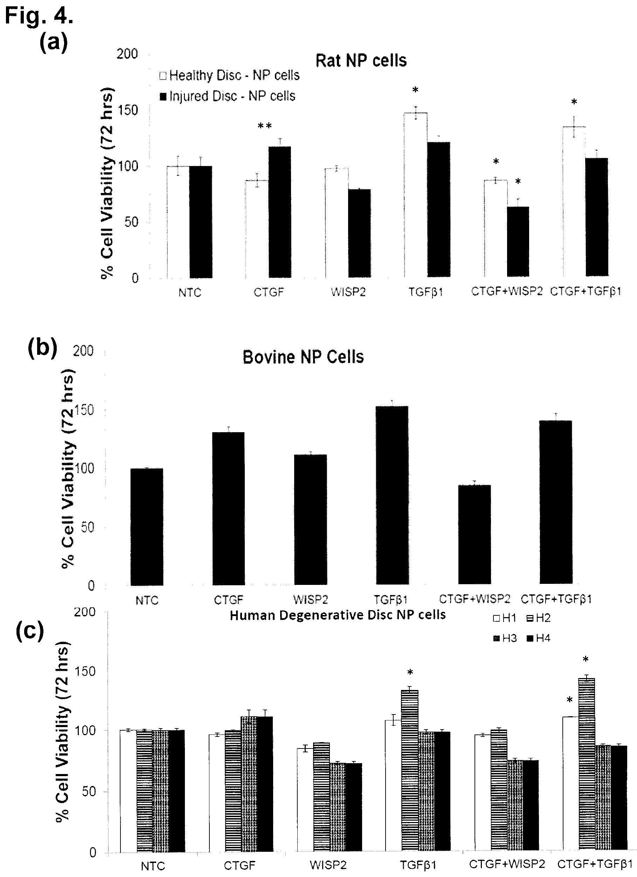

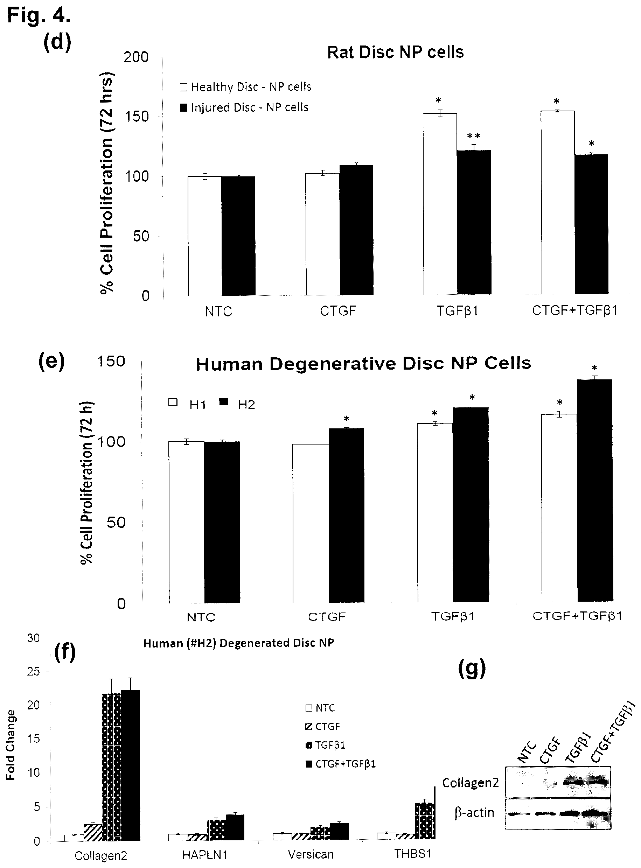

**FIG. 4** shows Anabolic effects of CTGF, WISP-2 and TGFβ1 in an *in vitro* model of DDD. (**a**) Effect of CTGF, WISP-2 and TGFβ1 treatment alone or in combination on cell viability (72 hrs) as determined using MTT assays in NP cells obtained from (**a**) rat tail (healthy / injured) discs **p=0.049, *p≤0.02), (**b**) bovine degenerative disc NPs, *p<0.01 and (**c)** human (H1- H4) degenerative disc NPs (*p<0.005). Each bar represents mean±S.D. of 3 independent experiments done in triplicates (n=9). Cell proliferation assays (72 hrs) in (**d**) rat NP cells (healthy / injured discs), *p<0.001, **p<0.01 and (**e**) human (H1, H2) degenerative disc NPs treated with CTGF and TGFβ1 alone or in combination as determined using colorimetric anti-BrdU - ELISA,*p<0.001. The p-values were determined using paired Student's t-test, for treatment with CTGF, WISP-2 or TGFβ1 alone or in combination with respect to no treatment control (NTC). (**f**) Histograms showing increased expression of collagen 2, HAPLN1, versican and thrombospondin1 on treatment of human degenerative disc NP cells with CTGF and TGFβ1 as revealed by real time PCR analysis. Each bar in the histogram represents the mean ± S.D. of 3 independent experiments done in duplicates (n=6,*p<0.001). (**g**) Western blot verifying increased collagen 2 expression in human degenerative disc NP cells on treatment with CTGF and TGFβ1.

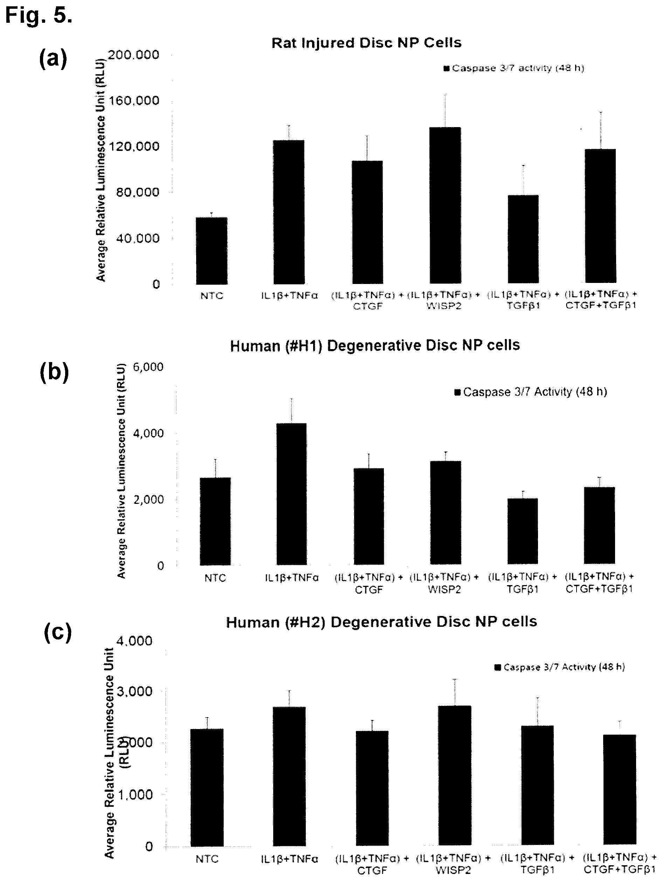

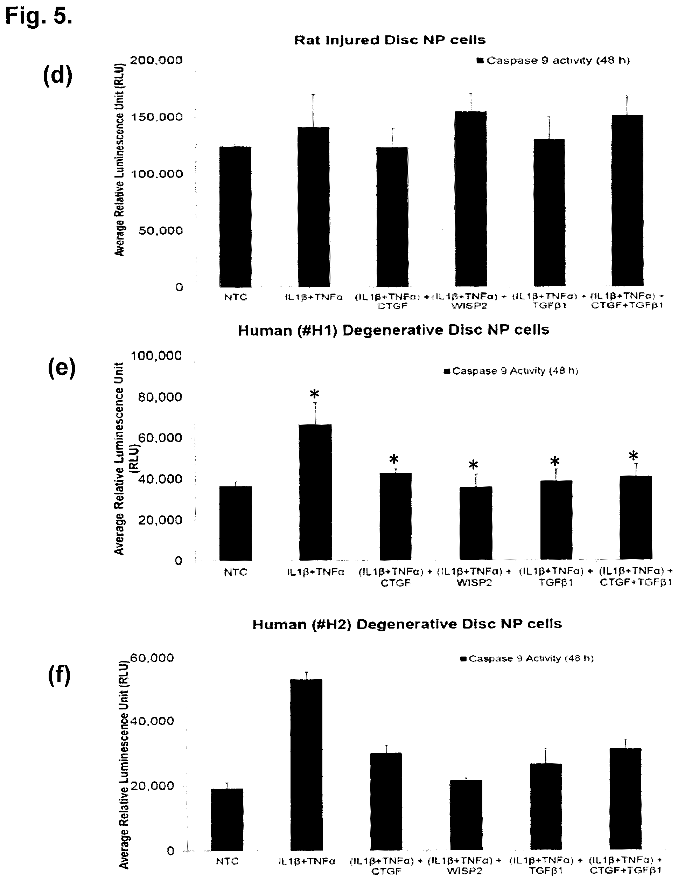

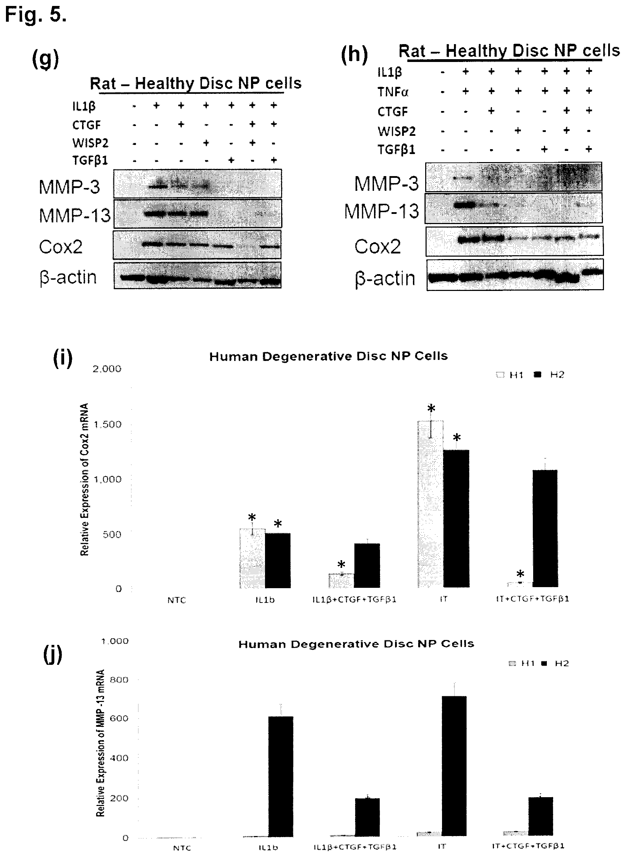

**FIG. 5** shows Anti-catabolic effects of CTGF, WISP-2 and TGFβ1 in an *in vitro* model of DDD. (**a**) Histograms showing caspase 3/7 activity in NP cells derived from (**a**) rat injured IVD (*p=0.008, **p=0.05), (**b, c**) human degenerative disc NP treated with pro-inflammatory cytokines, IL-1β and TNFα alone or in presence of CTGF, WISP-2 and TGFβ1(*p<0.05). Histograms showing caspase 9 activity in NP cells (**d**) rat injured IVD, (**e, f**) human degenerative disc NP treated with pro-inflammatory cytokines, IL-1β and TNFα alone or in presence of CTGF, WISP-2 and TGFβ1 (*p<0.05, **p<0.005). Each bar in the caspase assays is showing mean ± S.D. of 2 independent experiments done in quadruplets (n=8). The p-values were determined using paired Student's t-test. For the combination of IL-1β and TNFα are with respect to no treatment control (NTC), while p-values for the groups containing growth factors (CTGF, WISP-2 or TGFβ1) are with respect to the group containing combination of IL-1β and TNFα only. Western blot analysis of MMP-3, MMP-13 and Cox2 in rat healthy IVD NP cells treated with (**g**) IL-1β alone, (**h**) IL-1β and TNFα in combination, and in the presence of CTGF, WISP-2 or TGFβ1. Histograms showing decreased expression of (**i**) Cox2, (**j**) MMP-13 mRNA levels in human degenerative disc NP cells treated with IL-1β and TNFα in presence of CTGF and TGFβ1 in comparison to IL-1β and TNFα only treatments. Each bar in the histogram represents mean ± S.D. of 3 independent experiments done in duplicates (n=6,*p<0.001).

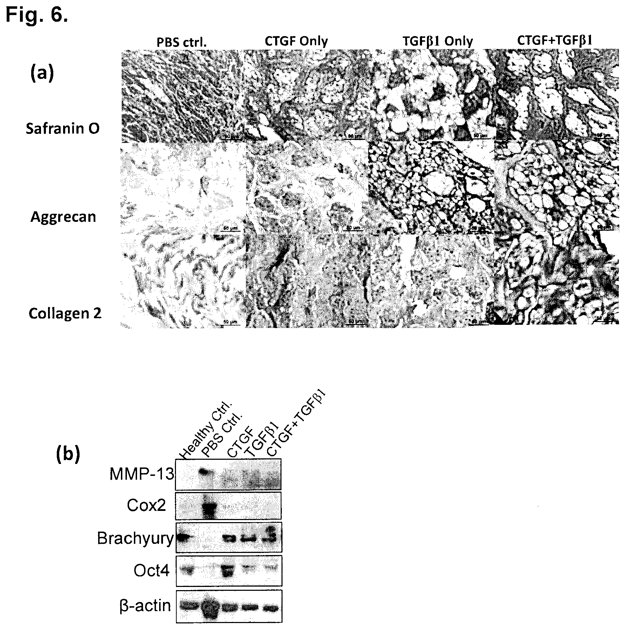

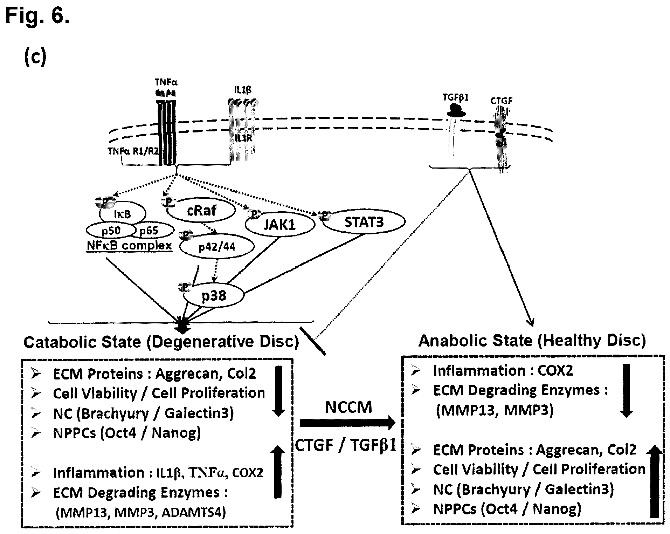

**FIG. 6** shows Evaluation of the regenerative potential of CTGF and TGFβ1 in a pre-clinical *in vivo* rodent disc injury model of DDD. (**a**) Representative Safranin-O and immunohistochemical staining of ECM proteins, aggrecan and collagen 2 in paraffin embedded sections of rat tail injured IVD - NPs treated with phosphate - buffered saline (PBS, used as a control), CTGF, TGFβ1 or a combination of CTGF and TGFβ1 (Scale bar 50µ). (**b**) Western blots showing decreased expression of MMP-13 and Cox2, and restoration of the NC marker, brachyury and stem cell marker, Oct4 in NP tissue lysates obtained from rat tail injured discs treated with CTGF, TGFβ1 or a combination of CTGF and TGFβ1. (**c**) Proposed model demonstrating the mechanism of progressive disc degeneration in presence of pro-inflammatory cytokines (IL-1β and TNFα) and effect of intervention by potential therapeutic agents (CTGF / TGFβ1) for regeneration of the IVD-NP.

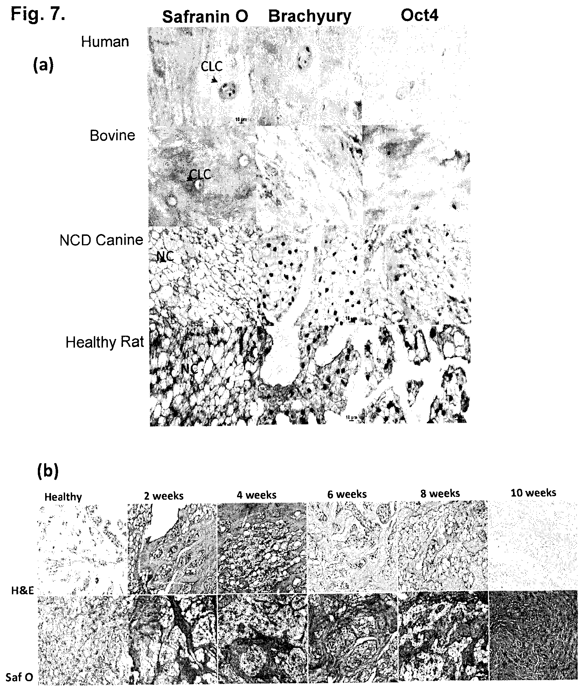

**FIG. 7** shows (a) Safranin O staining and immunohistochemical analysis of brachyury and Oct4 proteins in paraffin embedded sections of nucleus pulposus obtained from human degenerative disc, bovine degenerative disc, non-chondrodystrophic canine and healthy rat discs (Scale bar 10µ). Arrows represent CLC, chondrocyte like cells and NC, notochordal cells. (**b**) Histological changes in rat disc NP in a time dependent manner (healthy - 10 weeks post-injurypost-injury) with hematoxylin and eosin (H&E) staining showing loss of NCs and Safranin-O (SafO) staining showing development of fibrocartilaginous matrix.

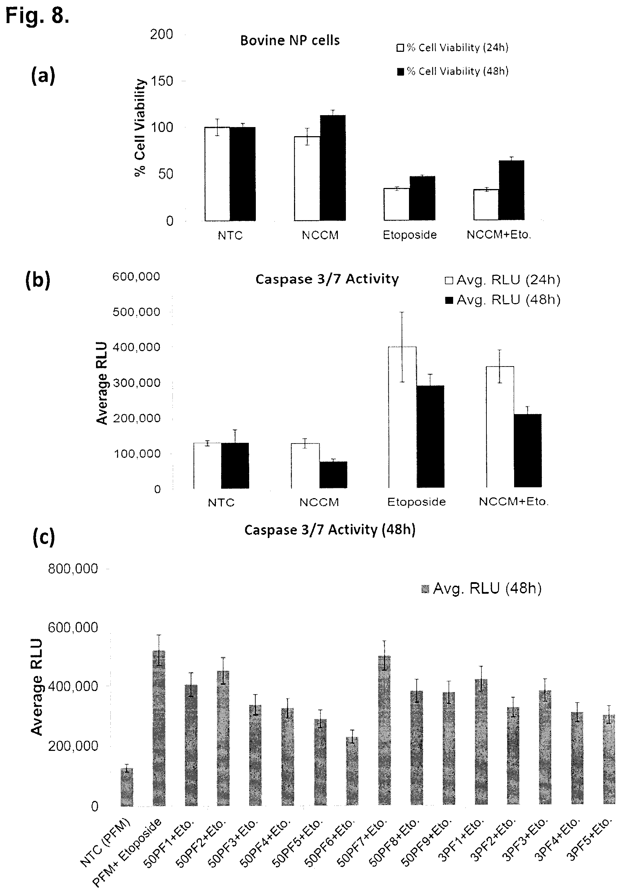

**FIG. 8** shows Evaluation of bioactivity in protein containing fractions (PF) collected after size exclusion chromatography. Histograms showing **(a)** cell viability, (**b**) caspase 3/7 activity in bovine NP cells treated with NCCM, the cytotoxic drug, etoposide (300 µM, used as a positive control) in presence of NCCM or control medium (serum, phenol red and protein free Hybridoma Media). Each bar represents mean ± S.D. of 2 independent experiments done in triplicates (n=6). (**c**) Histograms showing caspase 3/7 activity in bovine NP cells treated with etoposide in control medium (serum free, phenol red free Hybridoma Media) or in presence of protein containing fractions (PFs). Each bar represents mean ± S.D. of 2 independent experiments done in triplicates (n=6). For these experiments, protein containing fractions (PFs) were mixed (1:1) with control medium (serum free, phenol red free Hybridoma Media) and compared to no treatment controls (i.e. elution buffer + control medium (1:1).

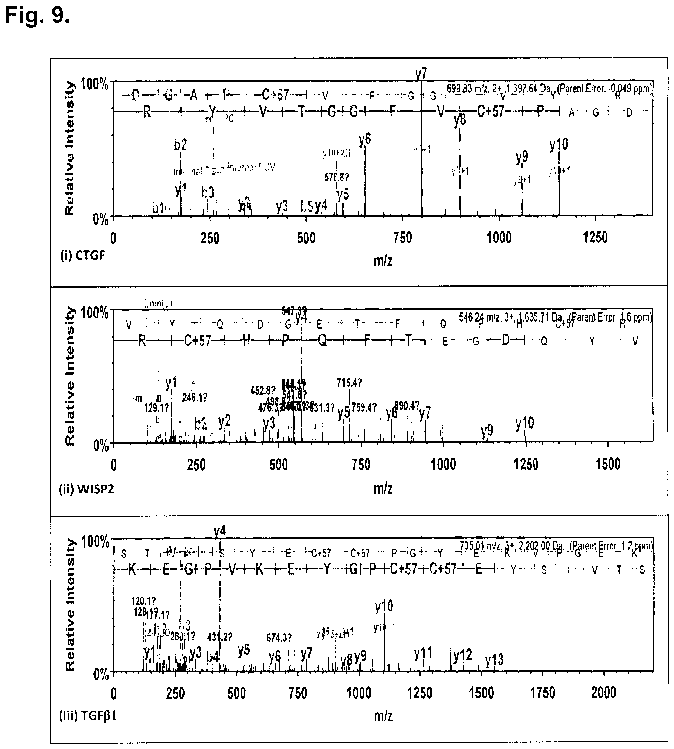

**FIG. 9** shows Peptide signature peaks for CTGF, WISP-2 and TGFβ1 observed in mass - spectroscopy analysis of NCCM.

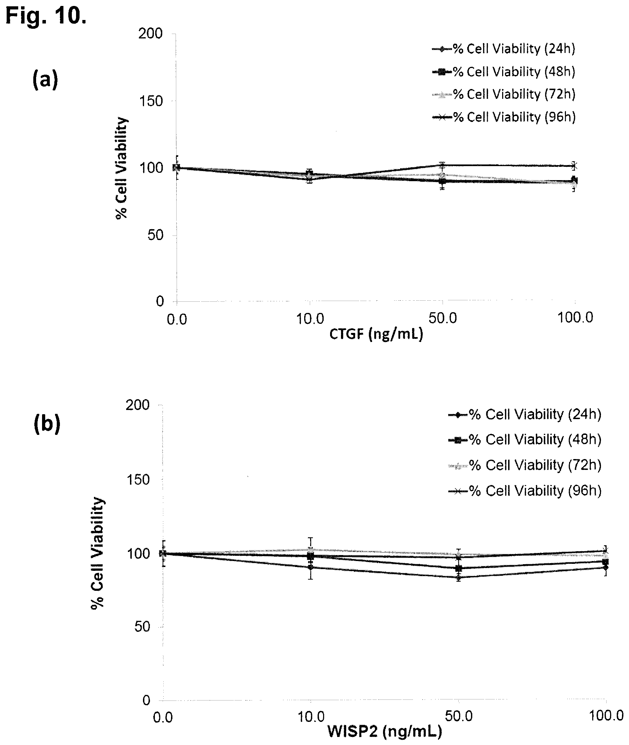

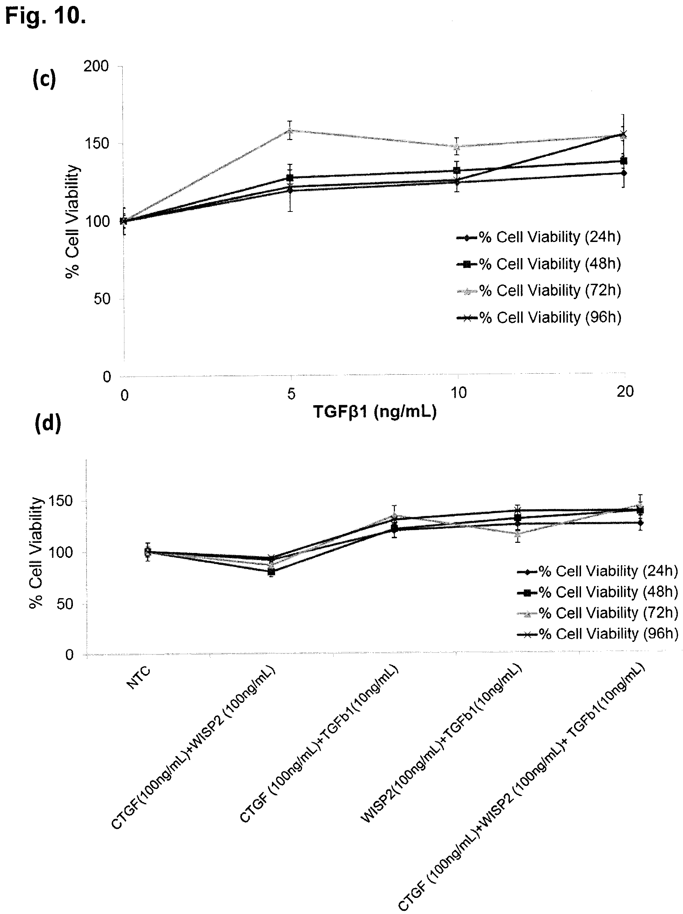

**FIG. 10** shows Dose (1 ng/mL - 100 ng/mL) and time dependent (24 hrs - 96 hrs) effects of (**a**) CTGF, (**b**) WISP-2 and (**c**) TGFβ1 treatment alone or (**d**) combination of CTGF with WISP-2, and TGFβ1 on cell viability determined using MTT assays in NP cells obtained from healthy rat IVDs. Each bar represents mean ± S.D. of 3 independent experiments done in quadruplicates (n=12).

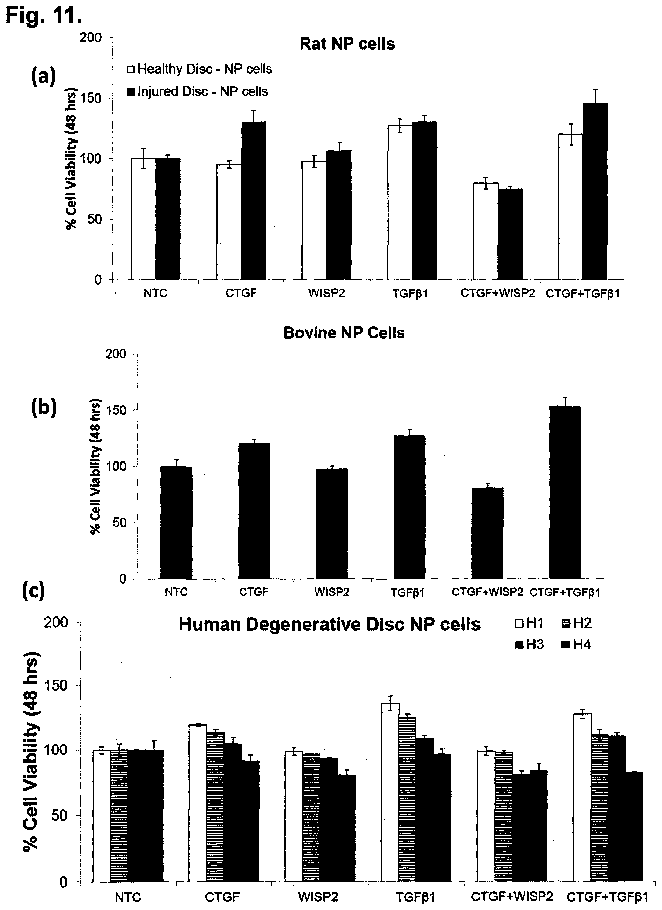

**FIG. 11** shows Effect of CTGF, WISP-2 and TGFβ1 treatment alone or in combination on cell viability as determined using MTT assays in NP cells obtained from (**a**) rat (healthy / injured) disc, *p≤0.02, (**b**) bovine degenerative disc, *p<0.01 and (**c**) human (H1- H4) degenerative NP cells in 48 hrs (*p<0.005,**p=0.02). Each bar represents mean ± S.D. of 3 independent experiments done in triplicates (n=9).

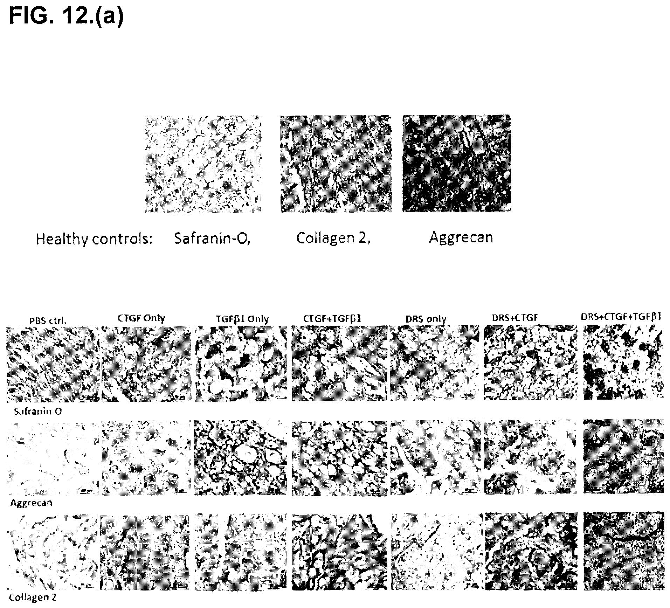

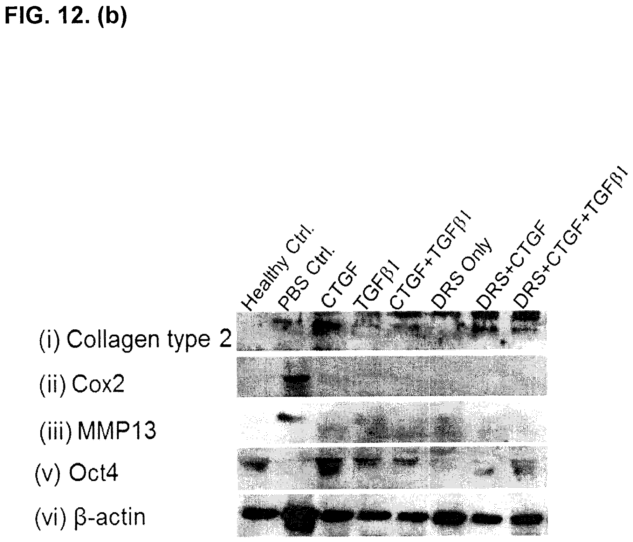

**FIG. 12** shows an evaluation of the regenerative potential of CTGF and TGFβ1 alone and in combination with DRS (solution comprising glucosamine hydrochloride and chondroitin sulphate ) in a pre-clinical in vivo rodent disc injury model of DDD, showing (a) Safranin-O and immunohistochemical staining of ECM proteins, aggrecan and collagen 2 in paraffin embedded sections of rat tail injured IVD - NPs with phosphate - buffered saline (PBS, used as a control) (Scale bar 50µ). (b) Western blots showing decreased expression of MMP-13 and Cox2, and restoration of the stem cell marker, Oct4 in NP tissue lysates obtained from rat tail injured discs treated with CTGF, TGFβ1, a combination of CTGF and TGFβ1, DRS, DRS in combination with CTGF or a combination of DRS, CTGF and TGFβ1.

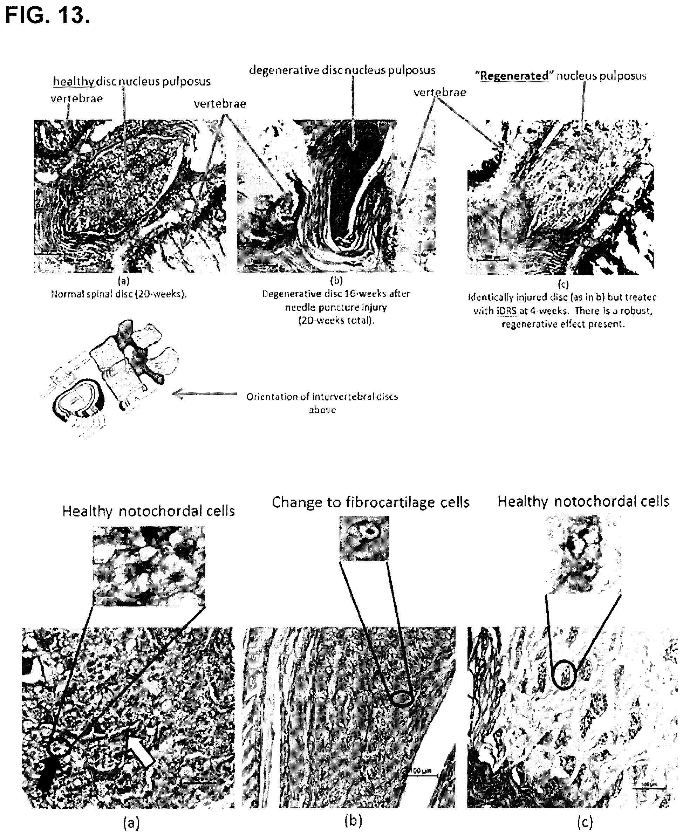

**FIG. 13** shows the effects of TGFβ1+CTGF+DRS compared to PBS injections 20-weeks post injury in a rat tail disc injury model. The PBS injected disc demonstrates fibrocartilaginous degenerative changes inclusive of fraying/tearing of the annulus fibrosus, loss of disc height and a degenerative change of the NP cell type. The TGFβ1+CTGF+DRS - injected disc (c) demonstrates a near normal appearing phenotype with preservation of a rich extracellular matrix, notochordal-appearing cells, sustained disc height and healthy annulus fibrosus.

### DETAILED DESCRIPTION

Embodiments of a composition according to the invention are described through reference to the drawings.

In an embodiment of the second aspect of the present disclosure, there is provided a composition suitable for intervertebral disc (IVD) injection comprising the following components in aqueous solution, or pharmaceutically acceptable salts thereof: chondroitin (preferably chondroitin sulphate), glucosamine (preferably glucosamine hydrochloride), one or both of connective tissue growth factor (CTGF) present at a concentration between 50 ng/mg to 500 ng/mg of the composition and transforming growth factor beta 1 (TGF-beta1) present at a concentration between 1 ng/mL and 100 ng/mL of the composition, and optionally further comprising dextrose, carboxymethylcellulose, dimethyl sulfoxide, and/or hyaluronic acid.

In some aspects of the present disclosure, a therapeutically effective amount of a composition for use in the treatment of pain emanating from a spinal disc, for use in the treatment of degenerative disc disease, for use in the treatment or prevention of spinal disc injury, for use in the regeneration of a degenerative spinal disc or for use in preventing the progression of loss of spinal disc height in a degenerative or injured spinal disc is for administering to the subject, by injection into a pain emanating spinal disc, a degenerative disc, a degenerative spinal disc, an injured spinal disc, or an adjacent spinal disc thereof.

As used herein, "therapeutically effective amount" refers to an amount effective, at dosages and for periods of time necessary, to achieve the desired therapeutic result. A therapeutically effective amount of the disclosed compositions may vary according to factors such as the disease state, age, sex, and weight of the individual, and the ability of the composition to elicit a desired response in the individual. A therapeutically effective amount is also one in which any toxic or detrimental effects are outweighed by the therapeutically beneficial effects.

Therapeutically effective compositions described herein suitably include between 1 to 100 ng/mL TGFβ1 or at least about 1, 5, 10, 15, 20, 25, 30, 35, 40, 45, 50, 55, 60, 65, 70, 75, 80, 90, 95, or 100 ng/mL TGFβ1. Therapeutically effective compositions described herein suitably include between 50 to 500 ng/mL CTGF or at least 50, 75, 100, 125, 150, 175, 200, 225, 250, 275, 300, 325, 350, 375, 400, 425, 450, 475, or 500 ng/mL CTGF. Therapeutic effects were seen in experiments utilizing 1 ng/mL TGFβ1, preferably 5 ng/mL TGFβ1, more preferably 10-100 ng/mL TGFB1; and in experiments utilizing at least about 50 ng/mL CTGF.

Compounds used in the compositions described herein can be used in the form of pharmaceutically acceptable salts derived from inorganic or organic acids. Pharmaceutically acceptable salt(s) are well-known in the art. For clarity, the term "pharmaceutically acceptable salts" as used herein generally refers to salts prepared from pharmaceutically acceptable non-toxic acids or bases including inorganic acids and bases and organic acids and bases. Suitable pharmaceutically acceptable base addition salts include metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art. See, e.g., Remington's Pharmaceutical Sciences, 18 th ed. (Mack Publishing, Easton Pa.: 1990) and Remington: The Science and Practice of Pharmacy, 19th ed. (Mack Publishing, Easton Pa.: 1995). The preparation and use of acid addition salts, carboxylate salts, amino acid addition salts, and zwitterion salts of compounds used in the compositions described herein may also be considered pharmaceutically acceptable if they are, within the scope of sound medical judgment, suitable for use in contact with the tissues of humans and lower animals without undue toxicity, irritation, allergic response, and the like, are commensurate with a reasonable benefit/risk ratio, and are effective for their intended use.

In a first aspect, there is provided a composition suitable for intervertebral disc (IVD) injection comprising CTGF present at a concentration between 50 ng/mg to 500 ng/mg of the composition and TGF-beta1 present at a concentration between 1 ng/mL and 100 ng/mL of the composition and at least one glycosaminoglycan, or a pharmaceutically acceptable salt thereof. In one embodiment, the at least one glycosaminoglycan comprises a glycosaminoglycan or derivative or precursor thereof. In one embodiment, the glycosaminoglycan or derivative or precursor thereof comprises chondroitin. In one embodiment, the glycosaminoglycan or derivative or precursor thereof comprises glucosamine. In one embodiment, the composition comprises CTGF, TGF-beta1, chondroitin and glucosamine.

In one embodiment, the composition further comprises at least one sugar. In one embodiment, the sugar is a cellulose derivative. In one embodiment, the sugar is carboxymethylcellulose. In one embodiment, the sugar is dextrose.

In one embodiment, the composition may comprise a further growth factor. In one embodiment, the further growth factor is WISP-2.

In one embodiment of the first aspect of the invention, there is provided a composition comprising chondroitin, glucosamine, CTGF present at a concentration between 50 ng/mg to 500 ng/mg of the composition, and TGF-β1 present at a concentration between 1 ng/mL and 100 ng/mL of the composition, or a pharmaceutically acceptable salt thereof. The composition suitably further includes water and dimethyl sulfoxide (DMSO), where the DMSO is up to 15%, up to 10% or preferably under about 5% based on the total composition weight. In one embodiment, the composition is a saline solution. In one embodiment, the composition further comprises dextrose or a pharmaceutically acceptable salt thereof.

In one embodiment, the composition further includes a buffer in a quantity sufficient to stabilize the composition at a desired pH. In one embodiment, the composition is stabilized at a pH between about 6 and about 7.

In one embodiment, the composition comprises chondroitin, preferably chondroitin sulfate.

In one embodiment, chondroitin, preferably chondroitin sulfate, is present at a level of about 0.1% to about 2.0%, in one embodiment, about 0.1% to about 1.0%, in another embodiment, 0.5 % to about 2% by weight based on the total composition.

In one embodiment, glucosamine, preferably glucosamine hydrochloride, is present at a level of about 1% to about 25%, in one embodiment about 1% to about 10%, in another embodiment about 5% to about 25% by weight based on the total composition.

In one embodiment, the composition further includes an anesthetic, suitably bupivacaine.

In one embodiment, the composition further includes a contrast agent, in one embodiment, a non-ionic contrast agent.

In one embodiment, dextrose is present at a level of ≤ about 25% by weight based on the total composition. In one embodiment, the dextrose is not present. In one embodiment, the dextrose is present at a level of ≤ about 5% by weight based on the total composition. In another embodiment, the dextrose is present at a level between about 1% and about 2% by weight based on the total composition.

The CTGF is present in the composition at a concentration between 50 and about 500 ng/mL of the composition, and is suitably present in the composition at a concentration of at least about 50 ng/mL of the composition, in one embodiment, at least about 100 ng/mL, in another embodiment, at least about 200 ng/mL.

The TGFβ 1 is present in the composition at a concentration between 1 and about 100 ng/mL of the composition, and is suitably present in the composition at a concentration of at least about 1 ng/mL of the composition, in one embodiment at least about 5 ng/mL, in another embodiment at least about 10 ng/mL.

In one embodiment, there is provided a composition that comprises, consists or consists essentially of:
chondroitin sulfate, present at a level of about 0.1% to about 2.0% by weight;
glucosamine hydrochloride, present at a level of about 1% to about 25 % by weight;
connective tissue growth factor, present at a concentration of about 50 ng/mg to about 500 ng/mg of the composition;
transforming growth factor beta 1, present at a concentration of about 10 ng/mg to about 100 ng/mg of the composition;
optionally, dextrose, at a level of about 0% to about 25% by weight;
optionally, carboxymethylcellulose, at a level of about 0% to about 0.5% by weight;
and an aqueous solution comprising water and optionally pharmaceutically acceptable carriers, buffer and/or dimethyl sulfoxide, at a collective level equaling the remainder of the composition by weight.

As illustrated in the Examples, compositions as described herein may suitably be used in therapy for disk degeneration or injury.

In one embodiment, there is provided a therapeutically effective amount of a composition as provided herein for use in the treatment of pain in a patient that emanates from a spinal disc, wherein the composition is for administration by injecting into the pain emanating disc. The disc may be a degenerated disc and/or a previously injured disc.

In one embodiment, there is provided a therapeutically effective amount of a composition as provided herein for use in a method of treating DDD in a patient, wherein the composition is for administration by injecting into the degenerative disc, or an adjacent spinal disc of the patient.
In one embodiment, there is provided a therapeutically effective amount of a composition as provided herein for use in in the treatment or prevention of spinal disc injury in a patient, wherein the composition is for administration by injecting into the injured spinal disc, or an adjacent spinal disc, of the patient.

While in one embodiment, the timing of treatment is not restricted, in one embodiment, treatment is performed within about 10 weeks following injury, and in other embodiments, within 4 weeks following injury, within 2 weeks following injury or within 96 hours following injury.

In one example not encompassed by the wording of the claims, there is provided a method of treating pain, arthritis, or suspected arthritis in a body area of a patient, the method comprising injecting a therapeutically effective amount of the composition described herein into the body area. The body area may be, but is not limited to, the spine or a joint. Other body areas that suitably may be treated according to methods described herein are the shoulder, wrist or elbow.

It will be understood that, where appropriate, treatment may include a prophylactic or preventative treatment, including treatment of an adjacent body area such as in an adjacent disc of the spine.

It will be appreciated by those skilled in the art that other variations of the embodiments described herein may also be practiced without departing from the scope of the invention. Other modifications are therefore possible.

Identified herein are factors secreted by notochordal cells (TGFβ1 and CTGF) that have the capacity to restore a healthy nucleus pulposus by altering the catabolic state of a degenerative intervertebral disc. Also shown, in multiple species including humans, is that the loss of both TGFβ1 and CTGF within the degenerative disc is associated with the development and progression of disc degeneration. The Examples demonstrate the utility of using a combination of TGFβ1 and CTGF in a novel, molecular regenerative therapy for DDD (see for example, Figs. 4(e), 5(i), and 5(j)). Moreover, there is additional utility in combining TGFβ1 and CTGF with therapeutic and other agents previously identified in U.S. Patent 8,048,865. In an embodiment, these agents comprise chondroitin, glucosamine, and dextrose. In an embodiment, these agents comprise a carrier, such as carboxymethylcellulose and/or hyaluronic acid. In an embodiment, the amount of carboxymethylcellulose present in a composition is less than about 0.5% by weight. The selection of appropriate pharmaceutically acceptable carriers depends on, among other things, the desired dosage form, body area to be impacted, and route of administration.

Although the disclosure has been described and illustrated in exemplary forms with a certain degree of particularity, it is noted that the description and illustrations have been made by way of example only. Numerous changes in the details of construction and combination and arrangement of parts and steps may be made. Accordingly, such changes are intended to be included in the invention, the scope of which is defined by the claims.
References to the methods of treatment in the examples of this description are to be interpreted as references to compositions of the present invention for use in those methods.

### Examples

Proteins present in bioactive fractions of NCCM using liquid chromatography and tandem mass spectroscopy (LC-MS/MS) were identified. The regenerative potential of TGFβ1, CTGF and Wnt-inducible soluble protein-2 (WISP2) identified in NCCM was evaluated using *in vitro* (rat, bovine and human NP cells) and a pre-clinical rodent model of DDD.

### Development of a preclinical model of DDD

The lack of a well characterized animal model of DDD poses a major challenge for comparative analysis and accurate assessment of potential therapeutic agents. Moreover, response to therapeutic agents are likely to be influenced by differences in histological and phenotypic differences among species^{25,26}. In search of an appropriate animal model that mimics human DDD and is suitable for evaluation of therapeutic agents, the histological characteristics of human degenerative disc NP was compared with bovine, NCD canine and Wistar rat IVDs. Strong Safranin-O staining demonstrative of a fibrocartilaginous matrix in NPs obtained from human and bovine degenerative IVDs (Fig. 7a) was observed. In contrast, healthy, notochordal cell-rich non-chondrodystrophic (NCD) - canines and young, healthy Wistar rats have a highly cellular NC - rich NP (>90%) with faint Safranin-O staining (Fig. 7a). Differences in cellular phenotype among NPs were verified using immunohistochemistry for brachyury, a NC-specific marker, and Oct4, a known marker of stem cells^{4,5}. Immunohistochemical analysis revealed no detectable expression of brachyury or Oct4 in human or bovine degenerative disc NPs (Fig. 7a). However, strong nuclear expression of brachyury and Oct4 was observed in healthy, young NCD canine and Wistar rat discs confirming the presence of NCs and stem cells within these NPs (Fig. 7a).

In order to establish a platform for the evaluation of therapeutic agents, a pre-clinical rodent model of DDD was adopted. Image-guided needle puncture injuries were perfomed in caudal (tail) discs of 12 week-old, healthy Wistar rats (n = 21, 4 discs per animal). Changes in the extra-cellular matrix (ECM) and the cellular phenotype were determined in a time dependent manner (72 hrs-10 weeks, Fig. 1a - g). Histological analysis revealed a gradual loss of NCs (>70%) along with increased Safranin-O staining intensity from 2 - 10 weeks, indicating ECM remodelling in the NP following injury (Fig. 1b, Fig. 7b). Decreased expression of aggrecan and collagen 2 was also observed by the end of 10 weeks post injury (Fig. 1b). Of note, needle puncture injury increased the expression of the pro-inflammatory cytokines, tumor necrosis factor alpha (TNFα) and interleukin-1 beta (IL-1β) as early as 72 hrs post-injury in NPs (Fig. 1c). However, the active form of IL-1β (~17 kDa) was not observed until 10 weeks, coincident with an abrupt increase in the expression of the inflammation mediator, cyclooxygenase 2 (COX2) and the ECM degrading enzymes, matrix metalloproteinases (MMP-3, MMP-13, Fig. 1c). Interestingly, the loss of tissue inhibitor of metalloproteinases 1 (TIMP1), a natural inhibitor of MMPs was observed at the end of 10 weeks post injury coincident with the onset of MMPs (Fig. 1c). A significant increase in the expression of A disintegrin-like and metalloprotease with thrombospondin type 1 motif 4 (ADAMTS4), one of the major enzymes responsible for aggrecan degradation was observed 1 week post-injury (Fig. 1c). In addition, needle puncture injury also induced phosphorylation of p42/44 (Thr202 / Tyr204) and p38MAPK (Thr180 / Tyr182) suggesting their role in disc degeneration (Fig. 1d). In parallel, western blotting and immunofluorescence using confocal microscopy demonstrated the loss of the NC markers (brachyury and galectin 3) and stem cell markers (Oct4 and Nanog) in the injured disc NPs, 10 weeks post-injury (Fig. 1e-g). These findings clearly demonstrated a shift in the NP *milieu* from a healthy, homeostatically regulated environment to a pro-inflammatory, catabolic state with loss of both NCs and stem cells in the degenerative disc.

### Regulation of ECM turnover in DDD

Unlike the hydrophilic, proteoglycan rich ECM seen in healthy NPs, the degenerative disc microenvironment is catabolic, rich in pro-inflammatory cytokines (IL-1β and TNFα) and reflects a failure of homeostasis as demonstrated in the rat-tail disc injury model. To determine the effect of IL-1β and TNFα on ECM turnover, rat NP cells were treated with IL-1β alone or in combination with TNFα for 24 hrs and real time quantitative PCR was performed using the ECM and cell adhesion molecule gene arrays (includes 84 genes). Treatment of rat NP cells with IL-1β alone or in combination with TNFα showed significant changes (p < 0.05) in the expression of 22 mRNA transcripts including downregulation of healthy matrix genes (HAPLN1, CTGF, Thrombospondin 1 and 2) and upregulation of matrix degrading enzymes, MMPs (MMP - 3 / 9 / 11 / 13) as compared to no treatment controls (NTC, Fig. 2a-c, Table 1):

**Table 1. List of Extra-cellular Matrix (ECM) Genes showing Upregulation / Downregulation on treatment with IL1beta alone / combination of IL1beta + TNFalpha w.r.t No treatment Control (NTC)**

| **Gene Symbol** | **Fold regulation (IL1β vs NTC)** | **p-value** | **Fold requlation (IL1β+TNFα vs NTC)** | **p-value** |
|---|---|---|---|---|
| **MMP9** | 1252.77 | **0.000** | 1218.76 | **0.000** |
| **Adamts8** | 139.39 | **0.000** | 81.95 | **0.000** |
| **MMP3** | 68.04 | **0.000** | 71.56 | **0.000** |
| **MMP13** | 36.82 | **0.005** | 42.39 | **0.000** |
| **Cd44** | 23.57 | **0.000** | 22.48 | **0.003** |
| **Itga3** | 922 | **0.000** | 7.04 | **0.003** |
| **Icam1** | 6.56 | **0.000** | 5.84 | **0.000** |
| **Itga5** | 6.23 | **0.000** | 8.12 | **0.034** |
| **Timp1** | 5.04 | **0.000** | 5.02 | **0.000** |
| **Emilin1** | 4.42 | **0.000** | 4.87 | **0.000** |
| **Col4a1** | 424 | **0.001** | 5.12 | **0.000** |
| **Lamc1** | 4.24 | **0.009** | 425 | **0.000** |
| **Col4a2** | 3.20 | **0.000** | 2.75 | **0.000** |
| **Itgav** | 2.14 | **0.000** | 2.22 | **0.004** |
| **Cdh3** | -6.18 | **0.410** | -8.58 | **0.035** |
| **Hapln1** | -6.01 | **0.001** | -12.59 | **0.000** |
| **Thbs2** | -4.14 | **0.001** | -5.90 | **0.000** |
| **Mmp11** | -3.86 | **0.004** | -6.51 | **0.001** |
| **Col3a1** | -3.80 | **0.000** | -3.53 | **0.000** |
| **Thbs1** | -3.35 | **0.000** | -2.87 | **0.000** |
| **Ctgf** | -2.40 | **0.001** | -2.71 | **0.000** |
| **Ncam1** | -2.00 | **0.001** | -3.68 | **0.000** |

Western blotting verified a marked increase in MMP-3 and MMP-13 expression levels in rat NP cells treated with IL-1β and TNFα (Fig. 2d). In addition, an increase in Cox2 but decreased Collagen 2 expression in rat NP cells was also observed in response to IL-1β and TNFα treatment (Fig. 2d).

Notably, IL-1β or its combination with TNFα increased phosphorylation levels of c-Raf (S259), p42/44 (Thr202 / Tyr204) and p38MAPK (Thr180 / Tyr182) without any significant change in their total protein content (Fig. 2d,e). Both IL-1β and TNFα failed to induce MMP-3, MMP-13 or Cox2 expression in presence of U0126, a specific inhibitor of p42/44MAPK in rat NP cells (Fig. 2f, g). In the presence of p38MAPK inhibitor, SB203580, reduced expression of MMP-3, MMP-13 and Cox2 was observed in IL-1β treated rat NP cells (Fig. 2f). These observations support *in vivo* data suggesting that the activation of p42/44 and p38MAPK downstream of IL-1β and TNFα are important in the regulation of ECM proteins during disc degeneration (Fig. 1d). The results obtained also suggest the involvement of nuclear factor kappa B (NFκB), Jun activated kinase 1 (JAK1), and the signal transducer and activation of transcription 3 (STAT3) in IL-1β and TNFα induced MMP-3, MMP-13 and Cox2 expression in NP cells. Reduced expression of MMP-3, MMP-13 and Cox2 proteins was shown in rat NP cells treated with IL-1β and TNFα in the presence of BAY-11-7082 (NFκB inhibitor,) JAK1 or STAT3 specific inhibitor (Fig. 2f, g). In contrast, presence of Wortamanin (PI3K inhibitor) reduced expression of MMP-3 and MMP-13 only (Fig. 2f, g). Similarly, in NP cells obtained from human degenerative discs, IL-1β and TNFα failed to induce Cox2 in presence of inhibitors of p38MAPK, NFκB, JAK1 or STAT3 (Fig. 2h). These findings suggested the importance of p38MAPK, NFκB, JAK1 and STAT3 proteins in progressive disc degeneration.

### NCCM promotes ECM turnover and reduces in vivo inflammation

The present inventors previously demonstrated that conditioned medium derived from notochordal cells (NCCM) showed anti-apoptotic effects, and induced upregulation in aggrecan and collagen 2 mRNA levels *in vitro*^{19,20}. However, the regenerative potential of NCCM in a pre-clinical *in vivo* model of DDD had not been evaluated. NCCM was collected by placing NC-rich NPs obtained from NCD-canines in phenol red free, serum free Hybridoma media and harvested conditioned medium according to previously described protocols¹⁸⁻²⁰. Concentrated NCCM or control medium (~8 µL/disc) was injected into the injured (4 week post-injury) rat-tail disc NPs using fluoroscopic imaging. Ten weeks post-injury, histological analysis revealed NC-rich NPs with moderate Safranin-O staining in NCCM injected rat-tail injured discs (Fig. 3a). In contrast, rat-tail injured discs injected with control medium showed low cellularity and displayed a fibrocartilaginous matrix, with intense Safranin-O staining, demonstrative of fibrocartilaginous morphology (Fig. 3a). Immunohistochemistry and western blotting revealed the restoration of aggrecan, collagen 2, brachyury, Oct4 and Nanog in rat-tail injured discs injected with NCCM in comparison to sham controls (Fig. 3a, b). These results demonstrated that soluble factors within NCCM have regenerative potential for DDD *in vivo.*

### Identification of soluble factors in NCCM using mass spectroscopy

To identify the soluble factors secreted by NCs, NCCM was concentrated sequentially using 50kDa and 3kDa filters, followed by fractionation using size exclusion chromatography (Fig. 3c). Among the protein containing fractions, only five fractions (50PF4, 50PF5, 50PF6, 3PF4 and 3PF5) reduced etoposide - induced caspase 3/7 activity in bovine NP cells (Fig. 8a - c). Mass spectroscopic analysis of these bioactive fractions led to the identification of 303 non-redundant proteins corresponding to the canine protein database (Fig. 3c).

Approximately 31% of these proteins had a secretory peptide signal sequence and have been reported within ECM (Fig. 3d). Growth factors and their modulators were identified including TGFβ1, connective tissue growth factor (CTGF), Wnt-induced soluble protein - 2 (WISP-2), chordin, sclerostin, cartilage intermediate layer protein (CILP) and CD109 (Fig. 9). Immunohistochemical analysis demonstrated moderate to strong immunostaining of CTGF, WISP-2 and TGFβ1 in the cytoplasm of NC cells and the ECM in healthy rat-tail disc NPs. However, injured rat-tail disc and human degenerated disc NPs showed no detectable expression of CTGF, WISP-2 or TGFβ1 within the ECM (Fig. 3e). These findings suggested that the loss of CTGF, WISP-2 or TGFβ1 is associated with the development of DDD.

### CTGF and TGFβ1 confers anabolic and anti-catabolic effects on NP cells in vitro

Rat-tail disc NP cells (healthy / degenerated) were treated with CTGF, WISP-2 or TGFβ1 to evaluate their effect on cell viability in a dose and time dependent manner (24 hrs - 96 hrs, Fig. 10a - d). Cell viability was determined using 3-(4,5-dimethylthiazol-2-yl)-2,5-diphenyltetrazolium bromide (MTT) based colorimetric assays. Treatment with CTGF (100 ng/ml) or TGFβ1 (10 ng/ml) alone or in combination increased the viability of NP cells derived from rat-tail discs (healthy / injured) and bovine discs in 48 hrs - 72 hrs (Fig. 4a, b, Fig. 11a, b). Notably, treatment with a combination of CTGF (100 ng/ml) and TGFβ1 (10 ng/ml) increased cell viability by ≥35% in human degenerated disc NP cells (Fig. 4c, Fig. 11c). However, no significant change in viability of NP cells (rat and human) was observed on treatment with WISP-2 (Fig. 4a-c, Fig. 10b, 5a-c). This was further confirmed with cell proliferation assays using bromodeoxyuridine (BrdU) incorporation. Significant increase in DNA synthesis was observed in rat-tail (healthy/injured) and human degenerated disc NP cells on treatment with TGFβ1 alone (Fig. 4d, e). Increased mRNA levels of collagen 2, hyaluronan and proteoglycan link protein 1 (HAPLN1), versican and thrombospondin1 (THBS1) were also observed in human degenerated disc NP cells on treatment with a TGFβ1 alone or in combination with CTGF (Fig. 4f). Western blotting verified an increase in collagen 2 expression on treatment with TGFβ1 alone or in combination with CTGF within 24 hrs (Fig. 4g), supporting the anabolic roles of these growth factors.
The potential of CTGF and TGFβ1 to suppress inflammation induced caspase activity and the expression of MMPs was evaluated. Both rat and human degenerative disc NP cells were treated with IL-1β alone or in combination with TNFα in the presence of CTGF, WISP-2 or TGFβ1 for 48 hrs. Results revealed a significant decrease in cytokine (IL-1β and TNFα)-induced caspase 3/7 activity in degenerative disc NP cells (rat / human) in the presence of TGFβ1 alone (Fig. 5a-c). In contrast, a significant reduction in IL-1β and TNFα induced caspase 9 activity was observed in the presence of either CTGF, WISP-2 or TGFβ1 in human degenerative disc NP cells (Fig. 5e,f). Treatment with CTGF, WISP-2 or TGFβ1 reduced expression of MMP-3, MMP-13 and Cox2 proteins in rat-tail NP cells treated with IL-1β and TNFα (Fig. 5g, h). Similarly, human degenerative disc NP cells treated with IL-1β and TNFα showed lower levels of Cox2 and MMP-13 mRNA levels in presence of a combination of CTGF and TGFβ1 demonstrating the anti-catabolic effect of these growth factors (Fig. 5i,j).

### Treatment with CTGF and TGFβ1 regenerates the degenerative disc nucleus pulposus in vivo

Results thus far suggested the anti-catabolic and pro-anabolic roles of CTGF and TGFβ1 in *in vitro.* To test the regenerative potential of CTGF and TGFβ1 in a rodent model, image guided tail disc injury (n = 30, 4 discs/animal) was performed in 2 independent experiments. Four weeks following injury, animals were randomized into 5 groups (n = 6 animals/group) and an intra-discal injection of CTGF (100 ng/mL), TGFβ1 (10 ng/mL), a combination of CTGF (100 ng/mL) and TGFβ1 (10 ng/mL) or phosphate buffered saline (PBS, 1X, pH = 7.2) as a vehicle control was given. Histological analysis of discs injected with PBS (1X, pH = 7.2) showed few cells with a fibrocartilaginous matrix and intense Safranin-O staining within the NP (Fig. 6a). However, injured rat-tail discs treated with CTGF or TGFβ1 alone or in combination, demonstrated a healthy disc, rich in NCs, 10 weeks post-injury (Fig. 6a). Immunohistochemical analysis confirmed the restoration of a healthy NP, showing strong expression of aggrecan and collagen II, as compared to injured disc NPs injected with vehicle control (Fig. 6a, b). Treatment with the combination of CTGF and TGFβ1 suppressed MMP-13 and Cox2 proteins, and restored expression of Brachyury and Oct4 in rat-tail injured disc NPs (Fig. 6b).

DDD is a multifactorial process characterized by the loss of IVD structural integrity, and the development of an inferior NP ECM often leading to a painful disc and limited mobility²⁷⁻³¹. In healthy IVD NPs, the hydrophilic ECM plays an important role in maintaining the biomechanical properties of the spine. The Examples show that deterioration of the NP - ECM in a rodent model of DDD is associated with inflammation, and the loss of notochordal and stem cells, which collectively lead to the development of a fibrocartilaginous NP, similar to that observed in human degenerative discs. These findings demonstrate a direct relationship between expression of pro-inflammatory cytokines (TNFα and IL-1β) and matrix degrading enzymes (MMP-3, MMP-13, ADAMTS4) in DDD. Treatment with these pro-inflammatory cytokines *in vitro* established a significant role for inflammation in the regulation of ECM degradation and turnover, during progressive disc degeneration. Therefore, targeting inflammation in a degenerative disc NP may be the key for treatment of DDD. Several single agent strategies have been designed and tested targeting inflammation in DDD, including an interleukin 1 receptor antagonist (IL-1Ra), as well as synthetic peptides and inhibitors targeting TNFα and its major downstream target, NFκB³²⁻³⁹. In support, it was also shown that inhibition of NFκB or MAPK (p42/44 and p38MAPK), Jak1 and STAT3 are capable of reducing Cox2, MMP-3 and MMP-13 expression downstream of IL-1β and TNFα in NP cells. However, the action of these specific inhibitors as therapeutic agents is limited to anti-catabolic activity. These agents fail to show any anabolic response that catalyzes *de novo* synthesis of healthy NP ECM or promote NP cell viability and proliferation in a degenerating IVD.

The present inventors identified TGFβ1, CTGF and WISP-2 in NCCM and demonstrated their regenerative potential. TGFβ1 plays a critical role in the development of the IVD and cartilage in the embryonic stage as well as post-natal development of the spine⁴⁰. Loss of TGFβ signaling in end plate chondrocytes and inner annulus fibrosus cells leads to the loss of matrix tissue, and abnormal growth plate morphology in the spines of TGFβ1-null mice⁴⁰. The data also showed reduced TGFβ1 expression in degenerated, fibrocartilaginous NP in both human and injured rat-tail discs. The loss of TGFβ1 in degenerative disc NPs indicates the importance of TGFβ signaling for the maintenance of a healthy, NC rich nucleus pulposus. TGFβ1 overexpression in healthy rabbit NPs showed a significant increase in proteoglycan synthesis in comparison to IVDs injected with control adenoviral vectors or saline⁴¹.

Another therapeutic agent, CTGF identified in NCCM is a matricellular protein that possesses an amino-terminal secretory peptide followed by four conserved domains with sequence homologies to insulin-like growth factor-binding proteins, von Willebrand factor C (VWC) domain, thrombospondin type 1 repeat (TSR) and a carboxy-terminal domain that contains a cysteine-knot motif. CTGF is an important constituent of the intervertebral disc microenvironment and interacts with several growth factors and matrix proteins including integrins and heparan sulfate proteoglycans. As shown in the Examples, treatment with the combination CTGF and TGFβ1 significantly reduced IL-1β induced expression of Cox2 and matrix degrading enzymes (MMP-3 and MMP-13), both in the *in vitro* and *in vivo* models of DDD, indicative of a combined action of these potential therapeutic agents (**Fig. 6c**).

### Regenerative potential of CTGF and TGFβ alone and in combination with DRS composition

FIG. 12(a) shows an evaluation of the regenerative potential of CTGF and TGFβ1 alone and in combination with DRS (a composition comprising glucosamine hydrochloride and chondroitin sulphate, prepared as under METHODS) in a *in vivo* rodent disc injury model of DDD described above. The figure shows Safranin-O and immunohistochemical staining of ECM proteins, aggrecan and collagen 2 with phosphate - buffered saline (PBS, used as a control). This evaluation demonstrated that the DRS + CTGF + TGFβ1 is a particularly effective treatment based on a comparison to healthy controls. FIG. 12(b) provides western blots showing decreased expression of MMP-13 and Cox2, and restoration of the stem cell marker, Oct4 in NP tissue lysates obtained from rat tail injured discs treated with CTGF, TGFβ1, a combination of CTGF and TGFβ1, DRS, DRS in combination with CTGF or a combination of DRS, CTGF and TGFβ1.

Fig. 13 shows further evaluation through 20 weeks in a rat-tail injury model of DDD: There are three different conditions present. (a) is a Safranin-O stained sagittal section of the rat-tail inter-vertebral disc (IVD) depicting a healthy nucleus pulposus, annulus fibrosus and vertebral end plates (red arrows). (b) Saf-O stained degenerative disc induced by needle puncture injury. The disc was injured as per normal methods followed 4-weeks later by an injection of PBS buffered saline and harvested 16 weeks post injection (20 weeks post injury). Results for the injury control are demonstrative of a profoundly degenerative phenotype with a distinct loss of height, tissue morphology, loss of notochordal cells and the development of a fibrocartilagenous extracellular matrix. (c) Identical experiment except 4-weeks post injury, a composition comprising CTGF, TGFβ1, and DRS was injected rather than saline. This treated disc reveals a near normal phenotype and morphology with maintenance of disc height, healthy vertebral endplates and sustained cellularity and healthy extracellular matrix.

### METHODS

Notochordal cell derived conditioned medium (NCCM) was collected from notochordal cell- rich nucleus pulpous (NP) obtained from IVDs of non-chondrodystrophic canines as described earlier¹⁸. All animals (n = 12) were obtained in collaboration with a licensed animal facility and all practices were in accordance with the animal care policies and ethics approval board of Toronto Western Hospital, Toronto, Ontario, Canada. All non-chondrodystrophic canines were 8 to 14 months of age and had failed at adoption or were to be euthanized for other purposes. Deep sedation was achieved using a combination of Acepromazine (10 mg/mL, Atravet-Aerst Pharmaceuticals St. Laurent, Quebec, Canada) mixed with Xylazine 100 mg/mL (Xylomax-Bimeda-NHC Animal Health, Broomhill Road, Tallaght, Dublin, Ireland) at a combined dose of 1 mL/15 Kg body weight. Once deep sedation had occurred, euthanasia was accomplished using intravenous sodium pentobarbital (CDMV) (St. Hyacinthe, Quebec, Canada) at a dose of 30 mL/kg body weight. Within 2 hrs of euthanization, the lumbar spines were removed and nucleus pulposus were isolated under aseptic conditions¹⁸. Nuclei pulposi were washed with phosphate buffered saline (PBS, pH = 7.2) and 2 - 3 NPs were placed within tissue culture inserts with 10µ-filters in CD Hybridoma media (protein and phenol red free, Cat No #11279-023, Life Technologies, USA) containing 100 units penicillin/streptomycin in 6 well plates under hypoxic conditions (3.5% O₂, and 5% CO₂, NuAire incubators) at 37⁰C. The conditioned medium referred hereafter as NCCM was collected after 24 hrs - 48 hrs, centrifuged at 8000 rpm for 30 minutes, filtered through 0.2µ syringe-tip filters and stored in -80⁰C until further use.

NCCM was thawed at room-temperature (RT) and concentrated sequentially using 50kDa and 3kDa spin-ultrafiltration protein concentrators (EMD Millipore, MA, USA) following manufacturer's instructions. The respective concentrated NCCM samples were fractionated by size-exclusion on a Superose 12 HR 10/30 fast protein liquid chromatography (FPLC) column (Pharmacia) in running buffer containing 10 mM sodium phosphate, 150 mM NaCl, 1 mM EDTA, pH 7.4. Thirty fractions (~1 ml) were collected, measured for protein concentration by absorbance at 280 nm and stored at -80⁰C until further use. Consecutive protein-containing fractions were pooled pairwise and evaluated for their effect on etoposide (cytotoxic drug) induced caspase 3/7 activity in bovine tail disc NP cells as described below. Bioactive fractions were defined as protein fractions showing a decrease in caspase 3/7 activity in bovine NP cells on treatment with etoposide. These bioactive fractions were later analyzed for identification of proteins using mass-spectroscopy.

Bioactive fractions were reduced with dithiothreitol (DTT), the free cysteine residues alkylated with iodoactetamide and digested overnight with modified bovine trypsin (Promega, Madison, USA). The tryptic peptides were desalted and loaded onto a 50 cm x 75 µm ID column containing RSLC 2 µm C18 packing material (EASY-Spray, Thermo-Fisher, Odense, Denmark) with an integrated emitter. The peptides were eluted into a Q-Exactive hybrid mass spectrometer (Thermo-Fisher, San Jose, CA) using an Easy-Spray nLC 1000 chromatography system (Thermo-Fisher, Odense Denmark) with a 90-minute gradient from 0% to 35% acetonitrile in 0.1% formic acid. The mass spectrometer was operated in a data dependent mode with 1 MS followed by 10 MS/MS spectra. The MS was acquired with a resolution of 70,000 FWHM, a target of 1 x 10⁶ ions and a maximum scan time of 120 ms. The MS/MS scans were acquired with a resolution of 17,500 FWHM, a target of 1 X 10⁶ ions and a maximum scan time of 120 ms using a relative collision energy of 27%. A dynamic exclusion time of 15 seconds was used for the MS/MS scans. The raw data files were acquired with XCalibur 2.2 (Thermo-Fisher Scientific) and processed with the Sequest search engine (Thermo-Fisher Scientific) using the UniProt canine database August 12, 2014 version with 28,460 entries and with X!-Tandem (Beavis Informatics, Winnipeg, MAN). The processed data was imported into Scaffold 3.2 (Proteome Software, Portland, OR). Peptides were considered to be identified if the Scaffold score exceeded the 0.1% false discovery rate (FDR) as determined by searching against the reversed UniProt canine database.

Bovine caudal disc NPs were obtained from six 3-year old steers. Human degenerative disc nucleus pulposus cells were obtained from patients (n=4) undergoing discectomy or fusion surgery at Toronto Western Hospital, University Health Network, Toronto, (with informed consent).

12-week old female Wistar rats (Charles River Laboratories International, Inc., MA, USA) were used in order to develop a pre-clinical rodent model of DDD and to evaluate the therapeutic potential of NCCM, CTGF and TGFβ1 in these pre-clinical rodent models. Experiments were performed in accordance with the Guide for the Care and Use of Laboratory Animals, and the experimental protocols were approved by the ethics approval board of Toronto Western Hospital, Toronto, ON, Canada. The surgical procedure was as follows: Anesthesia was achieved using isofluorane (5 L/min plus 1 L/min O₂) and maintained at 3 L/min. Once deeply anaesthetized, the animal was affixed on a stereotactic procedure apparatus (Model 900, Kopf Instruments California USA) with nose cone inhalation. For animal experiments, the tail was shaved and prepped with isopropanol in a sterile manner. Fluoroscopy was used to visualize needle penetration and to ensure that the needle penetrated into the center of the NP. For disc injury, a 26-gauge (G), 35⁰ beveled, 0.75 inches high needle (Hamilton Company, USA) mounted on a Hamilton syringe was used. The needle was advanced completely through the selected tail IVD to penetrate the full thickness inclusive of the annulus fibrosus on both sides of the disc. Confirmation of needle placement was made using fluoroscopy and maintained in position for 2 minutes, withdrawn halfway to the center of the NP and left there for 1 minute and then slowly withdrawn completely over a 1-minute period. The animals were then removed from the stereotactic apparatus and allowed to recover in a warmed cage. At the end of study period i.e. 72 hrs - 10 weeks, animals were humanely euthanized using CO₂ and each vertebral lumbar / caudal motion segment was dissected aseptically. IVDs were either fixed in formalin for histological analysis or the nucleus pulposus (healthy / injured) was harvested and lysed in RIPA buffer (50 mM Tris, pH = 7.4, 150 mM NaCl, 1% NP-40 and protease inhibitor cocktail) for western blotting.

To determine the regenerative potential of NCCM, CTGF or TGFβ1, 4 IVDs per animal of 12 week old rats were injured using a 26G needle as described above. Four weeks post-injury, animals were randomized into groups of six and an intra-discal injection (~8 µL) of either NCCM, CTGF (100 ng/mL) or TGFβ1 (10ng/mL) was given under local anesthesia. For NCCM injected animals, the control group consisted of animals that received an intra-discal injection (~8 µL) of Hybridoma culture medium only, while phosphate buffered saline ( PBS, 1X, pH = 7.2) served as control for CTGF and TGFβ1 injected animal groups. Six weeks later, the NPs from injured and control discs were harvested and either fixed in formalin for histological analysis or lysed in RIPA lysis buffer for western blotting. Each of this experiment was repeated independently to ensure reproducibility.

Cell Signaling Technology Sampler kits including rabbit polyclonal / monoclonal antibodies for phospho-p42/p44 (Thr202/Tyr204), phospho-p38 (Thr180/Tyr182), phospho-cRaf (Ser338), total p42/p44, p38MAPK proteins were obtained from New England Biolabs Ltd. (Ontario, Canada). Rabbit polyclonal antibodies for Collagen 2 (ab34712), MMP-13 (ab39012), Cox2 (ab15191), Oct4 (ab18976), Nanog (ab106465), CTGF (ab6995), STAT3 (ab7966), rabbit monoclonal for MMP-3 (ab52915) and mouse monoclonal antibodies for Galectin 3 (ab2785) and β-actin (ab6276) were purchased from Abcam Inc. (Toronto, Canada). Goat polyclonal Brachyury antibody (sc-17743) and rabbit polyclonal antibodies for Aggrecan (sc-25674), TIMP-1 (sc-5538), ADAMTS-4 (sc-25582), TNFα (sc-8301), TGFβ1 (sc-146) and mouse monoclonal WISP2 (sc-514070) were obtained from Santa Cruz Biotechnology Inc. (CA, USA). Specific inhibitors targeting p42/44 (U0126), p38MAPK (SB203580), NFκB (BAY-11-7082), PI3K (Wortamanin), JAK1 inhibitor and STAT3 inhibitor were purchased from EMD Millipore (Ontario, Canada). Human recombinant IL-1β, TNFα, CTGF, WISP2 and TGFβ1 proteins were purchased from Peprotech Inc. (Quebec, Canada).

The tissues were fixed in 10% formalin and decalcified in 10% EDTA solution. The decalcified disc was initially split in the mid sagittal plane, embedded in paraffin and 5µ thick sections were obtained for histological evaluation. Hemaetoxylin and eosin (H&E) and Safranin-O staining was performed to assess general morphology and proteoglycan content in these tissue sections as previously described¹⁶. For immunohistochemistry / immunofluorescence (IF), paraffin-embedded sections (5 µm) of human degenerative disc NP, bovine NP, healthy canines (NCD) and rat (healthy / injured) discs were deparaffinized in xylene, hydrated in gradient alcohol followed by antigen retrieval in Tris-EDTA buffer (pH = 9.0). The sections were incubated with hydrogen peroxide (0.3% v/v) in methanol for 15 minutes to quench the endogenous peroxidase activity, followed by blocking with 10% serum to preclude non-specific binding. Thereafter, the slides were incubated with either rabbit or goat polyclonal / mouse monoclonal primary antibodies overnight (O/N) at 4°C. Protein expression was detected using respective secondary antibodies (rabbit / goat / mouse) from Vectastain ABC kit and diaminobenzidine (DAB) as a chromogen. In negative controls, the primary antibody was replaced by isotype-matched IgG. The bright field sections were evaluated by light microscopic examination using a ScanScope XT, Aperio Whole Slide Scanner available at Advanced Optical Microscopy Facility (AOMF), Toronto Medical Discovery (TMDT). Images were analyzed using Aperio ImageScope (version 10). For immunofluorescence, primary antibodies were detected using Alexa fluor (488 / 568 nm) labeled respective secondary antibodies (rabbit / goat / mouse, Invitrogen, Life Technologies, CA, USA). The sections were counterstained with DAPI and mounted with Fluoromount (Sigma-Aldrich, USA) mounting media. All images were acquired using Fluoview 1000 inverted microscope (Olympus IX81, Olympus) available at AOMF, Toronto Medical Discovery (TMDT). Images were analyzed using Fluoview1000 (Version 3.1) software.

After euthanasia, healthy rat lumbar / caudal spine IVDs, injured tail discs and bovine caudal IVD NPs were removed aseptically and the nucleus pulposus (NP) was removed separately and enzymatically digested according to methods established by the present inventors¹⁶. Similarly, human degenerative disc NPs were enzymatically digested using Pronase (0.4%, 1 hr at 37°C) followed by Collagenase II treatment (0.015%, O/N, 37°C). The following day, the cells were filtered with a 70µ cell strainer and cultured within a hypoxic incubator (NuAire, MN, USA) in 3.5% O₂, 5% CO₂, in Advanced Dulbecco's modified Eagle's medium (ADMEM) supplemented with 8% fetal bovine serum (FBS) and penicillin and streptomycin (100 U/mL). For treatments, the cells were either cultured in serum free ADMEM (no treatment controls) or treated with interleukin-1β (IL-1β, 10 ng/mL), tumor necrosis factor-alpha (TNFα, 50 ng/mL), connective tissue growth factor (CTGF, 10 - 100ng/mL), Wnt-inducible soluble protein 2 (WISP2, 10 - 100ng/mL) or transforming growth factor beta 1 (TGFβ1, 5 - 20 ng/mL) for various time points under hypoxic conditions.

Equal amounts of whole cell or tissue lysates prepared using RIPA lysis buffer were subjected to Western blotting as described earlier^{18,19}. Total lysates (30 µg) were resolved on 10% sodium dodecyl sulphate-polyacrylamide gels (SDS-PAGE) under reducing conditions and then proteins were electro-transferred onto polyvinyledendifluoride (PVDF) membranes (BioRad, CA). After blocking with 5% non-fat powdered milk in Tris-buffered saline (TBS, 0.1 M, pH = 7.4), blots were incubated with rabbit or goat polyclonal / mouse monoclonal primary antibodies at 4°C overnight. Membranes were washed three times with Tween (0.1 %)-Tris-buffer saline (TTBS) and then incubated for 2 hrs at room temperature (RT) with the respective HRP-conjugated anti-IgG secondary antibodies (BioRad, CA), diluted as per the manufacturers suggestions in 2% non-fat milk in TBS (pH = 7.2, 1X). Blots were washed three times with TTBS for 15 minutes and protein bands were detected by the enhanced chemiluminescence method (BioRad, CA) on Kodak Hyperfilm.

Nucleus pulposus cells (rat, bovine and human) were plated in 96-well flat bottom plates in order to evaluate the effect of treatment with growth factors in cell viability and proliferation assays. Rat NP cells were treated with CTGF, WISP-2 and TGFβ1 in a dose (1 ng/mL - 100 ng/mL) and time dependent manner (24 hrs - 96 hrs) to determine the optimum dose and time for the assessment of these growth factors on viability. Cell viability was determined using 3-(4,5-dimethylthiazol-2-yl)-2,5-diphenyltetrazolium bromide (MTT, Sigma-Aldrich, USA) as previously described ⁴⁴. A BrdU - ELISA (colorimetric) assay (Cat# ab126556, Abcam) was used for determining the effect of treatment with CTGF or TGFβ1 on human and rat NP cells (healthy / injured) following the manufacturer's instructions. Briefly, NP cells were treated with CTGF or TGFβ1 alone or in combination for 48 hrs following addition of BrdU reagent O/N in each well. Incorporated BrdU in DNA of proliferating cells was determined using anti-BrdU antibody and quantified by ELISA as per the manufacturer's instructions.

Apoptosis induced in NP cells (rat and human) as result of treatment with pro-inflammatory cytokines (IL-1β and TNFα) was determined using Caspase 3/7 and Caspase 9 specific Lumi-Glo assays (Promega, Madison). Briefly, NP cells were plated and treated with either IL-1β alone or in combination with TNFα only or in presence of CTGF and TGFβ1 for 48 hrs, followed by addition of specific reagents for Caspase 3/7 and Caspase 9 as per the manufacturer's instructions. Cells were incubated for another 4 hrs at 37°C, and plates were read in a multi-well luminescence plate reader.

Total RNA from healthy and treated (IL-1β alone or in combination with TNFα) rat NP cells, were isolated using RNAeasy extraction kit (Cat#74134, Qiagen) and quantified using a Nanodrop spectrophotometer. Total RNA (~ 400ng) was reverse-transcribed using RT² First Strand Kit (Cat#330401, Qiagen) following the manufacturer's instructions for preparing cDNA. To evaluate the effect of IL-1β alone or in combination with TNFα on extra-cellular matrix (ECM) genes in rat NP cells, the RT² Profiler^{™} PCR Array Rat Extracellular Matrix & Adhesion Molecules (PARN-013Z, Qiagen) was used and compared with no treatment controls (NTC) in 3 independent experiments using real time PCR performed on ABI 7900HT 384-well Fast block machine. Data analysis including calculation of ΔΔCt values, fold changes and p-values was carried out using software available online (https://www.qiagen.com/ca). Total RNA was isolated from human degenerative disc NP cells (H1 / H2) treated with CTGF, TGFβ1, CTGF+TGFβ1 to evaluate their effect on collagen 2, HAPLN1, versican and thrombospondin 1 using gene specific primers. Similarly, RNA was isolated from human NP cells were treated with IL-1β alone or in combination TNFα in presence of CTGF and TGFβ1 to determine their effect on Cox2 and MMP-13 expression using qRT-PCR.

All data are expressed as means ± SD. Significant differences in test and no treatment controls were determined using the paired Student's t-test. Statistical analysis was performed using the Graphpad Prism. p < 0.05 was defined as statistically significant for all tests.

### Preparation of Exemplary Compositions

To a tube containing 30 ml 1X phosphate buffered saline (PBS) solution was added 0.048g of carboxymethylcellulose (cat# 419273, Sigma) by sprinkling. This tube was shaken for 2-3 hours at room temperature while wrapped in aluminum foil. 0.384 g of dextrose (cat #D8066, Sigma) was then added. 0.384 g of glucosamine hydrochloride (cat #G1514, Sigma) was then added. 72 mg of chondroitin sulphate (cat #C4384, Sigma) was then added, and any clumps of material were dispersed and dissolved using a 1 ml sterile plastic tip; this tube was then shaken until everything was dissolved. pH was adjusted with 1.0N NaOH to pH 7.4, forming a "DRS stock solution".

To make a "DRS working solution", a 1:10 dilution of DRS stock solution was made in 1X PBS, filtered through a 0.22 um syringe filter in a biosafety cabinet and stored in 4 degrees C.

To make a "CTGF stock solution", CTGF was purchased from Peprotech Inc. (cat# 120-19). 20 ug of lyophilized CTGF was centrifuged and dissolved with 1 ml of sterile deionized water.

To make a "CTGF working solution" in DRS, 5 ul of CTGF stock solution was added to 995 ul of DRS working solution resulting in 100 ng/ml of CTGF.

To make a "TGF-beta1 stock solution", TGF-beta1 was purchased from Peprotech Inc. (cat# 100-21). 10 ug of lyophilized TGF-beta1 was centrifuged and dissolved with 1 ml of sterile 10 mM citric acid (pH approx. 3.0).

To make a "CTGF + TGF-beta1 working solution in DRS", which had the most significant effects in the Figs. presented, 1 ul of the TGF-beta1 stock solution, 5 ul of the CTGF stock solution, and 994 ul of the DRS stock solution was mixed and aliquoted into four tubes of 250 ul each.

Although preferred embodiments of the invention have been described herein, it will be understood by those skilled in the art that variations may be made thereto without departing from the scope of the appended claims.

### References

1. Global Burden of Disease Study 2013 Collaborators. Global, regional, and national incidence, prevalence, and years lived with disability for 301 acute and chronic diseases and injuries in 188 countries, 1990-2013: a systematic analysis for the Global Burden of Disease Study 2013. Lancet 386, 743-800 (2015).
2. Hoy, D et al. The global burden of low back pain: estimates from the Global Burden of Disease 2010 study. Ann. Rheum. Dis. 73, 968-74 (2014).
3. Risbud MV, Schaer TP, Shapiro IM. Toward an understanding of the role of notochordal cells in the adult intervertebral disc: from discord to accord. Dev. Dyn. 239, 2141-8 (2010).
4. Risbud MV, Shapiro IM. Notochordal cells in the adult intervertebral disc: new perspective on an old question. Crit. Rev. Eukaryot. Gene. Expr. 21, 29-41 (2011).
5. Pattappa, G et al. Diversity of intervertebral disc cells: phenotype and function. J. Anat. 221, 480-96 (2012).
6. Phillips FM, Slosar PJ, Youssef JA, Andersson G, Papatheofanis F. Lumbar spine fusion for chronic low back pain due to degenerative disc disease: a systematic review. Spine (Phila Pa 1976). 38, E409-22 (2013).
7. Bydon, M et al. Lumbar fusion versus non-operative management for treatment of discogenic low back pain: a systematic review and meta-analysis of randomized controlled trials. J. Spinal. Disord. Tech. 27, 297-304 (2014).
8. Ren C, Song Y, Liu L, Xue Y. Adjacent segment degeneration and disease after lumbar fusion compared with motion-preserving procedures: a meta-analysis. Eur. J. Orthop. Surg. Traumatol. 24, S245-53 (2014).
9. Masuda K. Biological repair of the degenerated intervertebral disc by the injection of growth factors. Eur. Spine J. 17, 441-51 (2008).
10. Wang SZ, Rui YF, Lu J, Wang C. Cell and molecular biology of intervertebral disc degeneration: current understanding and implications for potential therapeutic strategies. Cell. Prolif. 47, 381-90 (2014).
11. Nishida, K et al. Gene therapy approach for disc degeneration and associated spinal disorders. Eur. Spine. J. 17, 459-66 (2008).
12. Wang SZ, Rui YF, Tan Q, Wang C. Enhancing intervertebral disc repair and regeneration through biology: platelet-rich plasma as an alternative strategy. Arthritis. Res. Ther. 15, 220 (2013).
13. Sakai D, Andersson GB. Stem cell therapy for intervertebral disc regeneration: obstacles and solutions. Nat. Rev. Rheumatol. 11, 243-56 (2015).
14. Wang, Z et al. Efficacy of intervertebral disc regeneration with stem cells - a systematic review and meta-analysis of animal controlled trials. Gene 564, 1-8 (2015).
15. Kandel R, Roberts S, Urban JP. Tissue engineering and the intervertebral disc: the challenges. Eur. Spine. J. 17, 480-91 (2008).
16. Bergknut, N et al. Intervertebral disc degeneration in the dog. Part 1: Anatomy and physiology of the intervertebral disc and characteristics of intervertebral disc degeneration. Vet. J. 195, 282-91 (2013).
17. Smolders, LA et al. Intervertebral disc degeneration in the dog. Part 2: chondrodystrophic and non-chondrodystrophic breeds. Vet. J. 195, 292-9 (2013).
18. Erwin WM, Ashman K, O'Donnel P, Inman RD. Nucleus pulposus notochord cells secrete connective tissue growth factor and up-regulate proteoglycan expression by intervertebral disc chondrocytes. Arthritis. Rheum. 54, 3859-67 (2006).
19. Erwin WM, Islam D, Inman RD, Fehlings MG, Tsui FW. Notochordal cells protect nucleus pulposus cells from degradation and apoptosis: implications for the mechanisms of intervertebral disc degeneration. Arthritis. Res. Ther. 13, R215 (2011).
20. Mehrkens, A et al. Canine notochordal cell-secreted factors protect murine and human nucleus pulposus cells from apoptosis by inhibition of activated caspase-9 and caspase-3/7. Evid. Based. Spine. Care. J. 4,154-6 (2013).
21. Bach, FC et al. The species-specific regenerative effects of notochordal cell-conditioned medium on chondrocyte-like cells derived from degenerated human intervertebral discs. Eur. Cell. Mater. 30, 132-47 (2015).
22. de Vries, SA et al. Conditioned medium derived from notochordal cell-rich nucleus pulposus tissue stimulates matrix production by canine nucleus pulposus cells and bone marrow-derived stromal cells. Tissue. Eng. Part. A. 21, 1077-84 (2015).
23. Korecki CL, Taboas JM, Tuan RS, latridis JC. Notochordal cell conditioned medium stimulates mesenchymal stem cell differentiation toward a young nucleus pulposus phenotype. Stem. Cell. Res. Ther. 1, 18 (2010).
24. Cornejo MC, Cho SK, Giannarelli C, latridis JC, Purmessur D. Soluble factors from the notochordal-rich intervertebral disc inhibit endothelial cell invasion and vessel formation in the presence and absence of pro-inflammatory cytokines. Osteoarthritis. Cartilage. 23, 487-96 (2015).
25. Singh K, Masuda K, An HS. Animal models for human disc degeneration. Spine. J. 5, 267S-279S (2005).
26. Alini, M et al. Are animal models useful for studying human disc disorders/degeneration? Eur. Spine. J. 17, 2-19 (2008).
27. Sivan, SS et al. Biochemical composition and turnover of the extracellular matrix of the normal and degenerate intervertebral disc. Eur. Spine. J. 23, S344-53 (2014).
28. Risbud MV, Shapiro IM. Role of cytokines in intervertebral disc degeneration: pain and disc content. Nat. Rev. Rheumatol. 10, 44-56 (2014).
29. Millward-Sadler SJ, Costello PW, Freemont AJ, Hoyland JA. Regulation of catabolic gene expression in normal and degenerate human intervertebral disc cells: implications for the pathogenesis of intervertebral disc degeneration. Arthritis. Res. Ther. 11, R65 (2009).
30. Vergroesen, PP et al. Mechanics and biology in intervertebral disc degeneration: a vicious circle. Osteoarthritis. Cartilage. 23, 1057-70 (2015).
31. Galbusera, F et al. Ageing and degenerative changes of the intervertebral disc and their impact on spinal flexibility. Eur. Spine. J. 23, S324-32 (2014).
32. Phillips KL, Jordan-Mahy N, Nicklin MJ, Le Maitre CL. Interleukin-1 receptor antagonist deficient mice provide insights into pathogenesis of human intervertebral disc degeneration. Ann. Rheum. Dis. 72, 1860-7 (2013).
33. Gorth, DJ et al. IL-1ra delivered from poly(lactic-co-glycolic acid) microspheres attenuates IL-1β-mediated degradation of nucleus pulposus in vitro. Arthritis. Res. Ther. 14, R179 (2012).
34. Goupille P, Mulleman D, Chevalier X. Is interleukin-1 a good target for therapeutic intervention in intervertebral disc degeneration: lessons from the osteoarthritic experience. Arthritis. Res. Ther. 9, 110 (2007).
35. Le Maitre CL, Hoyland JA, Freemont AJ. Interleukin-1 receptor antagonist delivered directly and by gene therapy inhibits matrix degradation in the intact degenerate human intervertebral disc: an in situ zymographic and gene therapy study. Arthritis. Res. Ther. 9, R83 (2007).
36. Le Maitre CL, Freemont AJ, Hoyland JA. A preliminary in vitro study into the use of IL-1Ra gene therapy for the inhibition of intervertebral disc degeneration. Int. J. Exp. Pathol. 87, 17-28 (2006).
37. Sinclair, SM et al. Attenuation of inflammatory events in human intervertebral disc cells with a tumor necrosis factor antagonist. Spine (Phila Pa 1976). 36, 1190-6 (2011).
38. Studer, RK et al. p38 MAPK inhibition in nucleus pulposus cells: a potential target for treating intervertebral disc degeneration. Spine (Phila Pa 1976). 32, 2827-33 (2007).
39. Cao, C et al. Bone marrow mesenchymal stem cells slow intervertebral disc degeneration through the NF-κB pathway. Spine. J. 15, 530-8 (2015).
40. Jin, H et al. TGF-β signaling plays an essential role in the growth and maintenance of intervertebral disc tissue. FEBS. Lett. 585, 1209-15 (2011).
41. Nishida, K et al. Modulation of the biologic activity of the rabbit intervertebral disc by gene therapy: an in vivo study of adenovirus-mediated transfer of the human transforming growth factor beta 1 encoding gene. Spine (Phila Pa 1976). 24, 2419-25 (1999).
42. Yang, H et al. TGF-β1 antagonizes TNF-α induced up-regulation of matrix metalloproteinase 3 in nucleus pulposus cells: role of the ERK1/2 pathway. Connect. Tissue. Res. 29, 1-8 (2015).
43. Tolonen, J et al. Growth factor expression in degenerated intervertebral disc tissue. An immunohistochemical analysis of transforming growth factor beta, fibroblast growth factor and platelet-derived growth factor. Eur. Spine. J. 15, 588-96 (2006).
44. Matta A, DeSouza LV, Ralhan R, Siu KW. Small interfering RNA targeting 14-3-3ζ increases efficacy of chemotherapeutic agents in head and neck cancer cells. Mol. Cancer. Ther. 9, 2676-88 (2010).

## Claims

1. A composition comprising:
at least one glycosaminoglycan, or a pharmaceutically acceptable salt thereof;
connective tissue growth factor (CTGF) present at a concentration between 50 ng/mg to 500 ng/mg of the composition; and
transforming growth factor beta 1 (TGFβ1) present at a concentration between 1 ng/mL and 100 ng/mL of the composition;
wherein the composition is suitable for intervertebral disc (IVD) injection.

2. A composition comprising:
chondroitin or a pharmaceutically acceptable salt thereof;
glucosamine or a pharmaceutically acceptable salt thereof; and
at least one growth factor selected from the group consisting of:
a) connective tissue growth factor (CTGF) present at a concentration between 50 ng/mg to 500 ng/mg of the composition; or
b) transforming growth factor beta 1 (TGFβ1) present at a concentration between 1 ng/mL and 100 ng/mL of the composition; or
c) CTGF present at a concentration between about 50 ng/mg to 500 ng/mg of the composition and TGFβ1 present at a concentration between 1 ng/mL and about 100 ng/mL of the composition;
wherein the composition is suitable for intervertebral disc (IVD) injection.

3. The composition of claim 1, comprising glucosamine or a pharmaceutically acceptable salt thereof or chondroitin or a pharmaceutically acceptable salt thereof.

4. The composition of claim 1, comprising glucosamine or a pharmaceutically acceptable salt thereof and chondroitin or a pharmaceutically acceptable salt thereof.

5. The composition of any one of claims 2 to 4 in which the chondroitin when present is chondroitin sulfate, optionally wherein the chondroitin sulfate is present at a level selected from the group consisting of 0.1% to 2.0% by weight of the composition, 0.5% to 2.0% by weight of the composition, 0.1% to 0.5% by weight of the composition, 0.1% by weight of the composition, and 0.5% by weight of the composition.

6. The composition of any one of claims 2 to 4 in which the glucosamine when present is glucosamine hydrochloride, optionally wherein the glucosamine hydrochloride is present at a level selected from the group consisting of 1% to 25% by weight of the composition, 5% to 20% by weight of the composition, 1% to 5% by weight of the composition, 1.0% to 1.5% by weight of the composition, 1% by weight of the composition, 1.5% by weight of the composition, 5% by weight of the composition, 20% by weight of the composition, and 25% by weight of the composition.

7. The composition of any one of claims 1 to 6 in which the CTGF when present is present at a concentration selected from the group consisting of: at least 50 ng/mL of the composition, at least 100 ng/mL of the composition, at least 200 ng/mL of the composition, between 50 ng/mg to 500 ng/mg of the composition, 50 ng/mL of the composition, 75 ng/mL of the composition, 100 ng/mL of the composition, 125 ng/mL of the composition, 150 ng/mL of the composition, 175 ng/mL of the composition, 200 ng/mL of the composition, 225 ng/mL of the composition, about 250 ng/mL of the composition, 275 ng/mL of the composition, 300 ng/mL of the composition, 325 ng/mL of the composition, 350 ng/mL of the composition, 375 ng/mL of the composition, 400 ng/mL of the composition, 425 ng/mL of the composition, 450 ng/mL of the composition, 475 ng/mL of the composition, and 500 ng/mL of the composition.

8. The composition of any one of claims 1 to 7 in which the TGFβ1 when present is present at a concentration selected from the group consisting of: at least 1 ng/mL of the composition, at least 5 ng/mL of the composition, at least 10 ng/mL of the composition, between 10 ng/mL and 100 ng/mL of the composition, between 10 ng/mg and 100 ng/mg of the composition, 1 ng/mL of the composition, 5 ng/mL of the composition, 10 ng/mL of the composition, 15 ng/mL of the composition, 20 ng/mL of the composition, 25 ng/mL of the composition, 30 ng/mL of the composition, 35 ng/mL of the composition, 40 ng/mL of the composition, 45 ng/mL of the composition, 50 ng/mL of the composition, 55 ng/mL of the composition, 60 ng/mL of the composition, 65 ng/mL of the composition, 70 ng/mL of the composition, 75 ng/mL of the composition, about 80 ng/mL of the composition, about 85 ng/mL of the composition, 90 ng/mL of the composition, 95 ng/mL of the composition, and 100 ng/mL of the composition.

9. The composition of any one of claims 1 to 8, further comprising at least one sugar, preferably wherein the at least one sugar is dextrose or a pharmaceutically acceptable salt thereof, preferably wherein the dextrose or the pharmaceutically acceptable salt thereof is present at a level selected from the group consisting of up to 25% by weight of the composition, preferably 1% to 2% by weight of the composition; and optionally when dependent upon claim 2 or claims 5 to 8 when dependent upon claim 2, the dextrose is present in about 1.0% to about 1.5% by weight of the composition.

10. The composition of claim 2, or any one of claims 5 to 9 when dependent on claim 2 further comprising at least one pharmaceutically acceptable carrier, optionally wherein the at least one pharmaceutically acceptable carrier is selected from the group consisting of carboxymethylcellulose and hyaluronic acid.

11. The composition of claim 1, 3 or 4 comprising carboxymethylcellulose.

12. The composition of claim 1, comprising chondroitin, glucosamine and dextrose, and optionally further comprising a carrier, optionally wherein the carrier is carboxymethylcellulose and/or hyaluronic acid and optionally wherein the carboxymethylcellulose is present at less than 0.5% by weight.

13. The composition of claim 2, or any one of claims 5 to 9 when dependent on claim 2, further comprising water.

14. The composition of claim 2, in the form of an aqueous solution, and optionally further comprising dextrose, carboxymethylcellulose, dimethyl sulfoxide and/or hyaluronic acid.

15. The composition of claim 2 or any one of claims 5 to 9 when dependent upon claim 2, further comprising a buffer, preferably wherein the buffer is present in a quantity sufficient to stabilize the composition pH between 6 and 7.

16. The composition of claim 2 or any one of any one of claims 5 to 9 when dependent upon claim 2, further comprising dimethyl sulfoxide.

17. The composition of claim 2 or any one of claims 5 to 9 when dependent upon claim 2, further comprising an anesthetic, preferably wherein the anesthetic is bupivacaine.

18. A therapeutically effective amount of the composition of any one of claims 1 to 17 for use in the treatment of pain in a patient that emanates from a spinal disc, preferably wherein the pain emanating spinal disc has degenerated or has been previously injured, and wherein the composition is for administration by injecting the composition into the pain emanating spinal disc, or an adjacent spinal disc, of the patient.

19. A therapeutically effective amount of the composition of any one of claims 1 to 17 for use in the treatment of degenerative disc disease in a patient, wherein the composition is for administration by injecting the composition into the degenerative disc, or an adjacent spinal disc, of the patient.

20. A therapeutically effective amount of the composition of any one of claims 1 to 17 for use in the treatment or prevention of spinal disc injury in a patient, wherein the composition is for administration by injecting the composition into the injured spinal disc, or an adjacent spinal disc, of the patient.

21. A therapeutically effective amount of the composition of any one of claims 2 or claims 5 to 9 when dependent on claim 2c for use in the regeneration of a degenerative spinal disc in a patient, wherein the composition is for administration by injecting the composition into the degenerative spinal disc, or an adjacent spinal disc, of the patient.

22. A therapeutically effective amount of the composition of claim 4 and any one of claims 5 to 12 when dependent on claim 4 or the composition of any one of claims 13 to 16 when dependent on claim 2 for use in preventing the progression of loss of spinal disc height in a degenerative or injured spinal disc of a patient, which degenerative or injured spinal disc has lost disc height relative to a non-degenerative or uninjured spinal disc, wherein the composition is for administration by injecting the composition into the degenerative or injured spinal disc, or an adjacent spinal disc, of the patient.

## Patentansprüche

1. Zusammensetzung, umfassend:
mindestens ein Glycosaminoglycan oder ein pharmazeutisch unbedenkliches Salz davon;
Connective tissue growth factor (CTGF), der in einer Konzentration zwischen 50 ng/mg bis 500 ng/mg der Zusammensetzung vorliegt; und
Transforming growth factor beta 1 (TGFβ1), der in einer Konzentration zwischen 1 ng/ml und 100 ng/ml der Zusammensetzung vorliegt;
wobei die Zusammensetzung zur Zwischenwirbelscheibeninjektion (IVD-Injektion) geeignet ist.

2. Zusammensetzung, umfassend:
Chondroitin oder ein pharmazeutisch unbedenkliches Salz davon;
Glucosamin oder ein pharmazeutisch unbedenkliches Salz davon; und
mindestens einen Wachstumsfaktor, der aus der Gruppe ausgewählt ist, die aus Folgendem besteht:
a) Connective tissue growth factor (CTGF), der in einer Konzentration zwischen 50 ng/mg bis 500 ng/mg der Zusammensetzung vorliegt; oder
b) Transforming growth factor beta 1 (TGFβ1), der in einer Konzentration zwischen 1 ng/ml und 100 ng/ml der Zusammensetzung vorliegt; oder
c) CTGF, der in einer Konzentration zwischen etwa 50 ng/mg bis 500 ng/mg der Zusammensetzung vorliegt, und TGFβ1, der in einer Konzentration zwischen 1 ng/ml und etwa 100 ng/ml der Zusammensetzung vorliegt;
wobei die Zusammensetzung zur Zwischenwirbelscheibeninjektion (IVD-Injektion) geeignet ist.

3. Zusammensetzung nach Anspruch 1, umfassend Glucosamin oder ein pharmazeutisch unbedenkliches Salz davon oder Chondroitin oder ein pharmazeutisch unbedenkliches Salz davon.

4. Zusammensetzung nach Anspruch 1, umfassend Glucosamin oder ein pharmazeutisch unbedenkliches Salz davon und Chondroitin oder ein pharmazeutisch unbedenkliches Salz davon.

5. Zusammensetzung nach einem der Ansprüche 2 bis 4, in der das Chondroitin, wenn es vorliegt, Chondroitinsulfat ist, wobei das Chondroitinsulfat optional in einem Anteil vorliegt, der aus der Gruppe ausgewählt ist, die aus 0,1 Gew.-% bis 2,0 Gew.-% der Zusammensetzung, 0,5 Gew.-% bis 2,0 Gew.-% der Zusammensetzung, 0,1 Gew.-% bis 0,5 Gew.-% der Zusammensetzung, 0,1 Gew.-% der Zusammensetzung und 0,5 Gew.-% der Zusammensetzung besteht.

6. Zusammensetzung nach einem der Ansprüche 2 bis 4, in der das Glucosamin, wenn es vorliegt, Glucosaminhydrochlorid ist, wobei das Glucosaminhydrochlorid optional in einem Anteil vorliegt, der aus der Gruppe ausgewählt ist, die aus 1 Gew.-% bis 25 Gew.-% der Zusammensetzung, 5 Gew.-% bis 20 Gew.-% der Zusammensetzung, 1 Gew.-% bis 5 Gew.-% der Zusammensetzung, 1,0 Gew.-% bis 1,5 Gew.-% der Zusammensetzung, 1 Gew.-% der Zusammensetzung, 1,5 Gew.-% der Zusammensetzung, 5 Gew.-% der Zusammensetzung, 20 Gew.-% der Zusammensetzung und 25 Gew.-% der Zusammensetzung besteht.

7. Zusammensetzung nach einem der Ansprüche 1 bis 6, in der der CTGF, wenn er vorliegt, in einer Konzentration vorliegt, die aus der Gruppe ausgewählt ist, die aus Folgendem besteht: mindestens 50 ng/ml der Zusammensetzung, mindestens 100 ng/ml der Zusammensetzung, mindestens 200 ng/ml der Zusammensetzung, zwischen 50 ng/mg bis 500 ng/mg der Zusammensetzung, 50 ng/ml der Zusammensetzung, 75 ng/ml der Zusammensetzung, 100 ng/ml der Zusammensetzung, 125 ng/ml der Zusammensetzung, 150 ng/ml der Zusammensetzung, 175 ng/ml der Zusammensetzung, 200 ng/ml der Zusammensetzung, 225 ng/ml der Zusammensetzung, etwa 250 ng/ml der Zusammensetzung, 275 ng/ml der Zusammensetzung, 300 ng/ml der Zusammensetzung, 325 ng/ml der Zusammensetzung, 350 ng/ml der Zusammensetzung, 375 ng/ml der Zusammensetzung, 400 ng/ml der Zusammensetzung, 425 ng/ml der Zusammensetzung, 450 ng/ml der Zusammensetzung, 475 ng/ml der Zusammensetzung und 500 ng/ml der Zusammensetzung.

8. Zusammensetzung nach einem der Ansprüche 1 bis 7, in der der TGFβ1, wenn er vorliegt, in einer Konzentration vorliegt, die aus der Gruppe ausgewählt ist, die aus Folgendem besteht: mindestens 1 ng/ml der Zusammensetzung, mindestens 5 ng/ml der Zusammensetzung, mindestens 10 ng/ml der Zusammensetzung, zwischen 10 ng/ml und 100 ng/ml der Zusammensetzung, zwischen 10 ng/mg und 100 ng/mg der Zusammensetzung, 1 ng/ml der Zusammensetzung, 5 ng/ml der Zusammensetzung, 10 ng/ml der Zusammensetzung, 15 ng/ml der Zusammensetzung, 20 ng/ml der Zusammensetzung, 25 ng/ml der Zusammensetzung, 30 ng/ml der Zusammensetzung, 35 ng/ml der Zusammensetzung, 40 ng/ml der Zusammensetzung, 45 ng/ml der Zusammensetzung, 50 ng/ml der Zusammensetzung, 55 ng/ml der Zusammensetzung, 60 ng/ml der Zusammensetzung, 65 ng/ml der Zusammensetzung, 70 ng/ml der Zusammensetzung, 75 ng/ml der Zusammensetzung, etwa 80 ng/ml der Zusammensetzung, etwa 85 ng/ml der Zusammensetzung, 90 ng/ml der Zusammensetzung, 95 ng/ml der Zusammensetzung und 100 ng/ml der Zusammensetzung.

9. Zusammensetzung nach einem der Ansprüche 1 bis 8, ferner umfassend mindestens einen Zucker, wobei der mindestens eine Zucker bevorzugt Dextrose oder ein pharmazeutisch unbedenkliches Salz davon ist, wobei die Dextrose oder das pharmazeutisch unbedenkliche Salz davon bevorzugt in einem Anteil vorliegt, der aus der Gruppe ausgewählt ist, die aus bis zu 25 Gew.-% der Zusammensetzung, bevorzugt 1 Gew.-% bis 2 Gew.-% der Zusammensetzung, besteht; und wobei die Dextrose optional, wenn abhängig von Anspruch 2 oder von den Ansprüchen 5 bis 8, wenn abhängig von Anspruch 2, in etwa 1,0 Gew.-% bis etwa 1,5 Gew.-% der Zusammensetzung vorliegt.

10. Zusammensetzung nach Anspruch 2 oder einem der Ansprüche 5 bis 9, wenn abhängig von Anspruch 2, ferner umfassend mindestens einen pharmazeutisch unbedenklichen Träger, wobei der mindestens eine pharmazeutisch unbedenkliche Träger optional ausgewählt ist aus der Gruppe bestehend aus Carboxymethylcellulose und Hyaluronsäure.

11. Zusammensetzung nach Anspruch 1, 3 oder 4, umfassend Carboxymethylcellulose.

12. Zusammensetzung nach Anspruch 1, umfassend Chondroitin, Glucosamin und Dextrose und optional ferner umfassend einen Träger, wobei der Träger optional Carboxymethylcellulose und/oder Hyaluronsäure ist und wobei optional die Carboxymethylcellulose mit weniger als 0,5 Gew.-% vorliegt.

13. Zusammensetzung nach Anspruch 2 oder einem der Ansprüche 5 bis 9, wenn abhängig von Anspruch 2, ferner umfassend Wasser.

14. Zusammensetzung nach Anspruch 2 in der Form einer wässrigen Lösung und optional ferner umfassend Dextrose, Carboxymethylcellulose, Dimethylsulfoxid und/oder Hyaluronsäure.

15. Zusammensetzung nach Anspruch 2 oder einem der Ansprüche 5 bis 9, wenn abhängig von Anspruch 2, ferner umfassend einen Puffer, wobei der Puffer bevorzugt in einem Umfang vorliegt, der ausreicht, um den pH-Wert der Zusammensetzung zwischen 6 und 7 zu stabilisieren.

16. Zusammensetzung nach Anspruch 2 oder einem der Ansprüche 5 bis 9, wenn abhängig von Anspruch 2, ferner umfassend Dimethylsulfoxid.

17. Zusammensetzung nach Anspruch 2 oder einem der Ansprüche 5 bis 9, wenn abhängig von Anspruch 2, ferner umfassend ein Anästhetikum, wobei das Anästhetikum bevorzugt Bupivacain ist.

18. Therapeutisch wirksame Menge der Zusammensetzung nach einem der Ansprüche 1 bis 17 zur Verwendung bei der Behandlung von Schmerz bei einem Patienten, der von einer Bandscheibe ausstrahlt, wobei bevorzugt die Schmerz ausstrahlende Bandscheibe degeneriert ist oder zuvor verletzt wurde und wobei die Zusammensetzung zur Verabreichung durch Injizieren der Zusammensetzung in die Schmerz ausstrahlende Bandscheibe oder eine benachbarte Bandscheibe des Patienten dient.

19. Therapeutisch wirksame Menge der Zusammensetzung nach einem der Ansprüche 1 bis 17 zur Verwendung bei der Behandlung von degenerativer Bandscheibenerkrankung bei einem Patienten, wobei die Zusammensetzung zur Verabreichung durch Injizieren der Zusammensetzung in die degenerative Bandscheibe oder eine benachbarte Bandscheibe des Patienten dient.

20. Therapeutisch wirksame Menge der Zusammensetzung nach einem der Ansprüche 1 bis 17 zur Verwendung bei der Behandlung oder Prävention einer Bandscheibenverletzung bei einem Patienten, wobei die Zusammensetzung zur Verabreichung durch Injizieren der Zusammensetzung in die verletzte Bandscheibe oder eine benachbarte Bandscheibe des Patienten dient.

21. Therapeutisch wirksame Menge der Zusammensetzung nach einem der Ansprüche 2 oder der Ansprüche 5 bis 9, wenn abhängig von Anspruch 2c, zur Verwendung bei der Regeneration einer degenerativen Bandscheibe bei einem Patienten, wobei die Zusammensetzung zur Verabreichung durch Injizieren der Zusammensetzung in die degenerative Bandscheibe oder eine benachbarte Bandscheibe des Patienten dient.

22. Therapeutisch wirksame Menge der Zusammensetzung nach Anspruch 4 und einem der Ansprüche 5 bis 12, wenn abhängig von Anspruch 4, oder der Zusammensetzung nach einem der Ansprüche 13 bis 16, wenn abhängig von Anspruch 2, zur Verwendung bei der Prävention des Fortschreitens eines Verlusts der Bandscheibenhöhe bei einer degenerativen oder verletzten Bandscheibe eines Patienten, wobei die degenerative oder verletzte Bandscheibe relativ zu einer nicht degenerativen oder unverletzten Bandscheibe an Bandscheibenhöhe verloren hat, wobei die Zusammensetzung zur Verabreichung durch Injizieren der Zusammensetzung in die degenerative oder verletzte Bandscheibe oder eine benachbarte Bandscheibe des Patienten dient.

## Revendications

1. Composition comprenant :
au moins un glycosaminoglycane, ou un sel acceptable pharmaceutiquement de celui-ci ;
un facteur de croissance du tissu conjonctif (CTGF) présent à une concentration comprise entre 50 ng/mg et 500 ng/mg de la composition ; et
un facteur de croissance transformant bêta 1 (TGFβ1) présent à une concentration comprise entre 1 ng/ml et 100 ng/ml de la composition ;
dans laquelle la composition convient à une injection dans un disque intervertébral (IVD).

2. Composition comprenant :
de la chondroïtine ou un sel acceptable pharmaceutiquement de celle-ci ;
de la glucosamine ou un sel acceptable pharmaceutiquement de celle-ci ; et
au moins un facteur de croissance choisi dans le groupe constitué de :
a) un facteur de croissance du tissu conjonctif (CTGF) présent à une concentration comprise entre 50 ng/mg et 500 ng/mg de la composition ; ou
b) un facteur de croissance transformant bêta 1 (TGFβ1) présent à une concentration comprise entre 1 ng/ml et 100 ng/ml de la composition ; ou
c) un CTGF présent à une concentration comprise entre environ 50 ng/mg et 500 ng/mg de la composition et un TGFβ1 présent à une concentration comprise entre 1 ng/ml et environ 100 ng/ml de la composition ;
dans laquelle la composition convient à une injection dans un disque intervertébral (IVD).

3. Composition de la revendication 1, comprenant de la glucosamine ou un sel acceptable pharmaceutiquement de celle-ci ou de la chondroïtine ou un sel acceptable pharmaceutiquement de celle-ci.

4. Composition de la revendication 1, comprenant de la glucosamine ou un sel acceptable pharmaceutiquement de celle-ci et de la chondroïtine ou un sel acceptable pharmaceutiquement de celle-ci.

5. Composition de l'une quelconque des revendications 2 à 4, dans laquelle la chondroïtine, lorsqu'elle est présente, est le sulfate de chondroïtine, éventuellement dans laquelle le sulfate de chondroïtine est présent à un taux choisi dans le groupe constitué de 0,1 % à 2,0 % en poids de la composition, 0,5 % à 2,0 % en poids de la composition, 0,1 % à 0,5 % en poids de la composition, 0,1 % en poids de la composition et 0,5 % en poids de la composition.

6. Composition de l'une quelconque des revendications 2 à 4, dans laquelle la glucosamine, lorsqu'elle est présente, est le chlorhydrate de glucosamine, éventuellement dans laquelle le chlorhydrate de glucosamine est présent à un taux choisi dans le groupe constitué de 1 % à 25 % en poids de la composition, 5 % à 20 % en poids de la composition, 1 % à 5 % en poids de la composition, 1,0 % à 1,5 % en poids de la composition, 1 % en poids de la composition, 1,5 % en poids de la composition, 5 % en poids de la composition, 20 % en poids de la composition et 25 % en poids de la composition.

7. Composition de l'une quelconque des revendications 1 à 6, dans laquelle le CTGF, lorsqu'il est présent, est présent à une concentration choisie dans le groupe constitué de : au moins 50 ng/ml de la composition, au moins 100 ng/ml de la composition, au moins 200 ng/ml de la composition, entre 50 ng/mg et 500 ng/mg de la composition, 50 ng/ml de la composition, 75 ng/ml de la composition, 100 ng/ml de la composition, 125 ng/ml de la composition, 150 ng/ml de la composition, 175 ng/ml de la composition, 200 ng/ml de la composition, 225 ng/ml de la composition, environ 250 ng/ml de la composition, 275 ng/ml de la composition, 300 ng/ml de la composition, 325 ng/ml de la composition, 350 ng/ml de la composition, 375 ng/ml de la composition, 400 ng/ml de la composition, 425 ng/ml de la composition, 450 ng/ml de la composition, 475 ng/ml de la composition et 500 ng/ml de la composition.

8. Composition de l'une quelconque des revendications 1 à 7, dans laquelle le TGFβ1, lorsqu'il est présent, est présent à une concentration choisie dans le groupe constitué de : au moins 1 ng/ml de la composition, au moins 5 ng/ml de la composition, au moins 10 ng/ml de la composition, entre 10 ng/ml et 100 ng/ml de la composition, entre 10 ng/mg et 100 ng/mg de la composition, 1 ng/ml de la composition, 5 ng/ml de la composition, 10 ng/ml de la composition, 15 ng/ml de la composition, 20 ng/ml de la composition, 25 ng/ml de la composition, 30 ng/ml de la composition, 35 ng/ml de la composition, 40 ng/ml de la composition, 45 ng/ml de la composition, 50 ng/ml de la composition, 55 ng/ml de la composition, 60 ng/ml de la composition, 65 ng/ml de la composition, 70 ng/ml de la composition, 75 ng/ml de la composition, environ 80 ng/ml de la composition, environ 85 ng/ml de la composition, 90 ng/ml de la composition, 95 ng/ml de la composition et 100 ng/ml de la composition.

9. Composition de l'une quelconque des revendications 1 à 8, comprenant en outre au moins un sucre, de préférence dans laquelle l'au moins un sucre est le dextrose ou un sel acceptable pharmaceutiquement de celui-ci, de préférence dans laquelle le dextrose ou le sel acceptable pharmaceutiquement de celui-ci est présent à un taux choisi dans le groupe constitué de jusqu'à 25 % en poids de la composition, de préférence de 1 % à 2 % en poids de la composition ; et éventuellement lorsqu'elle dépend de la revendication 2 ou des revendications 5 à 8 lorsqu'elles dépendent de la revendication 2, le dextrose est présent à raison d'environ 1,0 % à environ 1,5 % en poids de la composition.

10. Composition de la revendication 2, ou de l'une quelconque des revendications 5 à 9 lorsqu'elles dépendent de la revendication 2, comprenant en outre au moins un support acceptable pharmaceutiquement, éventuellement dans laquelle l'au moins un support acceptable pharmaceutiquement est choisi dans le groupe constitué de la carboxyméthylcellulose et de l'acide hyaluronique.

11. Composition de l'une des revendications 1, 3 ou 4, comprenant de la carboxyméthylcellulose.

12. Composition de la revendication 1, comprenant de la chondroïtine, de la glucosamine et du dextrose, et comprenant éventuellement en outre un support, éventuellement dans laquelle le support est la carboxyméthylcellulose et/ou l'acide hyaluronique et éventuellement dans laquelle la carboxyméthylcellulose est présente à raison de moins de 0,5 % en poids.

13. Composition de la revendication 2, ou de l'une quelconque des revendications 5 à 9 lorsqu'elles dépendent de la revendication 2, comprenant en outre de l'eau.

14. Composition de la revendication 2, sous la forme d'une solution aqueuse, et comprenant éventuellement en outre du dextrose, de la carboxyméthylcellulose, du diméthylsulfoxyde et/ou de l'acide hyaluronique.

15. Composition de la revendication 2 ou de l'une quelconque des revendications 5 à 9 lorsqu'elles dépendent de la revendication 2, comprenant en outre un tampon, de préférence dans laquelle le tampon est présent en une quantité suffisante pour stabiliser le pH de la composition entre 6 et 7.

16. Composition de la revendication 2 ou de l'une quelconque des revendications 5 à 9 lorsqu'elles dépendent de la revendication 2, comprenant en outre du diméthylsulfoxyde.

17. Composition de la revendication 2 ou de l'une quelconque des revendications 5 à 9 lorsqu'elles dépendent de la revendication 2, comprenant en outre un anesthésique, de préférence dans laquelle l'anesthésique est la bupivacaïne.

18. Quantité thérapeutiquement efficace de la composition de l'une quelconque des revendications 1 à 17, destinée à être utilisée dans le traitement de la douleur chez un patient, laquelle émane d'un disque rachidien, de préférence dans laquelle le disque rachidien à l'origine de la douleur est dégénéré ou a précédemment subi une lésion, et dans laquelle la composition est destinée à être administrée par injection de la composition dans le disque rachidien à l'origine de la douleur ou dans un disque rachidien adjacent, du patient.

19. Quantité thérapeutiquement efficace de la composition de l'une quelconque des revendications 1 à 17, destinée à être utilisée dans le traitement d'une discopathie dégénérative chez un patient, dans laquelle la composition est destinée à être administrée par injection de la composition dans le disque dégénératif ou dans un disque rachidien adjacent du patient.

20. Quantité thérapeutiquement efficace de la composition de l'une quelconque des revendications 1 à 17, destinée à être utilisée dans le traitement ou la prévention d'une lésion d'un disque rachidien chez un patient, dans laquelle la composition est destinée à être administrée par injection de la composition dans le disque rachidien lésé ou dans un disque rachidien adjacent du patient.

21. Quantité thérapeutiquement efficace de la composition de l'une quelconque des revendications 2 ou des revendications 5 à 9 lorsqu'elles dépendent de la revendication 2c, destinée à être utilisée dans la régénération d'un disque rachidien dégénératif chez un patient, dans laquelle la composition est destinée à être administrée par injection de la composition dans le disque rachidien dégénératif ou dans un disque rachidien adjacent, du patient.

22. Quantité thérapeutiquement efficace de la composition de la revendication 4 et de l'une quelconque des revendications 5 à 12 lorsqu'elles dépendent de la revendication 4 ou de la composition de l'une quelconque des revendications 13 à 16 lorsqu'elles dépendent de la revendication 2, destinée à être utilisée dans la prévention de la progression de la perte de hauteur de disque rachidien dans un disque rachidien dégénératif ou lésé d'un patient, lequel disque rachidien dégénératif ou lésé a perdu de la hauteur de disque par rapport à un disque rachidien non dégénératif ou non lésé, dans laquelle la composition est destinée à être administrée par injection de la composition dans le disque rachidien dégénératif ou lésé ou dans un disque rachidien adjacent, du patient.
